# EUROPEAN PATENT APPLICATION

(11) **EP 4 063 506 A1**
(43) Date of publication of application: **28.09.2022**
(21) Application number: 20866578.6
(22) Date of filing: 18.09.2020
(51) Int. Cl.: C12N 15/13, G01N 33/573, A61K 39/395, A61P 3/06

(54) **METHOD FOR PREVENTING OR TREATING CHOLESTEROL-RELATED DISEASES BY USING ANTI-PCSK9 ANTIBODY**

(30) Priority: 19.09.2019 CN 201910884902; 19.05.2020 CN 202010424274
(71) Applicant: Innovent Biologics (Suzhou) Co., Ltd., Suzhou, Jiangsu 215123 (CN)
(72) Inventor: QIAN, Lei, Suzhou, Jiangsu 215123 (CN); ZHENG, Shirui, Suzhou, Jiangsu 215123 (CN); DENG, Huan, Suzhou, Jiangsu 215123 (CN)
(74) Representative: Ruscoe, Peter James
(86) International application number: PCT/CN2020/116245
(87) International publication number: WO 2021/052472

(57) **Abstract**

The present invention relates to use of an anti-PCSK9 antibody and/or antibody fragment thereof and a pharmaceutical preparation comprising the same in a method for preventing or treating a cholesterol-related disease. Furthermore, the present invention relates to an anti-PCSK9 antibody or a liquid preparation thereof for preventing or treating the above disease.

SEQUENCE LISITNG

<110>  Innovent Biologics (Suzhou) Co., Ltd.

<120>  USE OF ANTI-PCSK9 ANTIBODY IN METHOD FOR PREVENTING OR TREATING
CHOLESTEROL-RELATED DISEASE

<130>  PF 200572PCT

<160> 10

<170>  PatentIn version 3.3

<210>  4
<211>  11
<212>  PRT
<213>  Artificial Sequence

<220>
<223>  Light chain variable region CDR1

<400>  4

Arg Ala Ser Gln Ser Val Ser Ser Tyr Leu Ala
1               5                   10

<210>  5
<211>  7
<212>  PRT
<213>  Artificial Sequence

<220>
<223>  Light chain variable region CDR2

<400>  5

Asp Ala Ser Asn Arg Ala Thr
1               5

<210>  6
<211>  8
<212>  PRT
<213>  Artificial Sequence

<220>
<223>  Light chain variable region CDR3

<400>  6

Gln Gln Arg Arg Asn Trp Phe Thr
1               5

<210>  10
<211>  11
<212>  PRT
<213>  Artificial Sequence

<220>
<223>  Heavy chain variable region CDR1

<400>  10

Gly Ser Ile Ser Ser Ala Ser Tyr Tyr Trp Ser
1               5                   10

<210>  17
<211>  16
<212>  PRT
<213>  Artificial Sequence

<220>
<223>  Heavy chain variable region CDR2

<400>  17

Ser Ile Asn Tyr Arg Gly Ser Thr Tyr Tyr Asn Pro Ser Leu Lys Ser
1               5                   10                  15

<210>  19
<211>  18
<212>  PRT
<213>  Artificial Sequence

<220>
<223>  Heavy chain variable region CDR3

<400>  19

Ala Arg Glu Asn Ser Gly Val Val Pro Ala Ala Gly Pro Asn Trp Phe
1               5                   10                  15

Gly Pro

<210>  24
<211>  106
<212>  PRT
<213>  Artificial Sequence

<220>
<223> Light chain variable region

<400>  24

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Arg Asn Trp Phe Thr
                85                  90                  95

Phe Gly Gly Gly Thr Lys Val Glu Ile Lys
            100                 105

<210>  30
<211>  126
<212>  PRT
<213>  Artificial Sequence

<220>
<223>  Heavy chain variable region of ADI-10087

<400>  30

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Ala
            20                  25                  30

Ser Tyr Tyr Trp Ser Trp Ile Arg Gln Pro Pro Gly Lys Gly Leu Glu
        35                  40                  45

Trp Ile Gly Ser Ile Asn Tyr Arg Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Glu Asn Ser Gly Val Val Pro Ala Ala Gly Pro Asn Trp
            100                 105                 110

Phe Gly Pro Trp Gly Gln Gly Thr Leu Val Thr Val Ser Ser
        115                 120                 125

<210>  35
<211>  213
<212>  PRT
<213>  Artificial Sequence

<220>
<223>  Light chain

<400>  35

Glu Ile Val Leu Thr Gln Ser Pro Ala Thr Leu Ser Leu Ser Pro Gly
1               5                   10                  15

Glu Arg Ala Thr Leu Ser Cys Arg Ala Ser Gln Ser Val Ser Ser Tyr
            20                  25                  30

Leu Ala Trp Tyr Gln Gln Lys Pro Gly Gln Ala Pro Arg Leu Leu Ile
        35                  40                  45

Tyr Asp Ala Ser Asn Arg Ala Thr Gly Ile Pro Ala Arg Phe Ser Gly
    50                  55                  60

Ser Gly Ser Gly Thr Asp Phe Thr Leu Thr Ile Ser Ser Leu Glu Pro
65                  70                  75                  80

Glu Asp Phe Ala Val Tyr Tyr Cys Gln Gln Arg Arg Asn Trp Phe Thr
                85                  90                  95

Phe Gly Gly Gly Thr Lys Val Glu Ile Lys Arg Thr Val Ala Ala Pro
            100                 105                 110

Ser Val Phe Ile Phe Pro Pro Ser Asp Glu Gln Leu Lys Ser Gly Thr
        115                 120                 125

Ala Ser Val Val Cys Leu Leu Asn Asn Phe Tyr Pro Arg Glu Ala Lys
    130                 135                 140

Val Gln Trp Lys Val Asp Asn Ala Leu Gln Ser Gly Asn Ser Gln Glu
145                 150                 155                 160

Ser Val Thr Glu Gln Asp Ser Lys Asp Ser Thr Tyr Ser Leu Ser Ser
                165                 170                 175

Thr Leu Thr Leu Ser Lys Ala Asp Tyr Glu Lys His Lys Val Tyr Ala
            180                 185                 190

Cys Glu Val Thr His Gln Gly Leu Ser Ser Pro Val Thr Lys Ser Phe
        195                 200                 205

Asn Arg Gly Glu Cys
    210

<210>  41
<211>  451
<212>  PRT
<213>  Artificial Sequence

<220>
<223>  Heavy chain of ADI-10087

<400>  41

Gln Leu Gln Leu Gln Glu Ser Gly Pro Gly Leu Val Lys Pro Ser Glu
1               5                   10                  15

Thr Leu Ser Leu Thr Cys Thr Val Ser Gly Gly Ser Ile Ser Ser Ala
            20                  25                  30

Ser Tyr Tyr Trp Ser Trp Ile Arg Gln Pro Pro Gly Lys Gly Leu Glu
        35                  40                  45

Trp Ile Gly Ser Ile Asn Tyr Arg Gly Ser Thr Tyr Tyr Asn Pro Ser
    50                  55                  60

Leu Lys Ser Arg Val Thr Ile Ser Val Asp Thr Ser Lys Asn Gln Phe
65                  70                  75                  80

Ser Leu Lys Leu Ser Ser Val Thr Ala Ala Asp Thr Ala Val Tyr Tyr
                85                  90                  95

Cys Ala Arg Glu Asn Ser Gly Val Val Pro Ala Ala Gly Pro Asn Trp
            100                 105                 110

Phe Gly Pro Trp Gly Gln Gly Thr Leu Val Thr Val Ser Ser Ala Ser
        115                 120                 125

Thr Lys Gly Pro Ser Val Phe Pro Leu Ala Pro Cys Ser Arg Ser Thr
    130                 135                 140

Ser Glu Ser Thr Ala Ala Leu Gly Cys Leu Val Lys Asp Tyr Phe Pro
145                 150                 155                 160

Glu Pro Val Thr Val Ser Trp Asn Ser Gly Ala Leu Thr Ser Gly Val
                165                 170                 175

His Thr Phe Pro Ala Val Leu Gln Ser Ser Gly Leu Tyr Ser Leu Ser
            180                 185                 190

Ser Val Val Thr Val Pro Ser Ser Asn Phe Gly Thr Gln Thr Tyr Thr
        195                 200                 205

Cys Asn Val Asp His Lys Pro Ser Asn Thr Lys Val Asp Lys Thr Val
    210                 215                 220

Glu Arg Lys Cys Cys Val Glu Cys Pro Pro Cys Pro Ala Pro Pro Val
225                 230                 235                 240

Ala Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Gln Phe Asn Trp Tyr Val Asp Gly Val Glu
        275                 280                 285

Val His Asn Ala Lys Thr Lys Pro Arg Glu Glu Gln Phe Asn Ser Thr
    290                 295                 300

Phe Arg Val Val Ser Val Leu Thr Val Val His Gln Asp Trp Leu Asn
305                 310                 315                 320

Gly Lys Glu Tyr Lys Cys Lys Val Ser Asn Lys Gly Leu Pro Ala Pro
                325                 330                 335

Ile Glu Lys Thr Ile Ser Lys Thr Lys Gly Gln Pro Arg Glu Pro Gln
            340                 345                 350

Val Tyr Thr Leu Pro Pro Ser Arg Glu Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Met Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Gly
    450

## Description

The present application claims the benefits of the Chinese Patent Application No. 201910884902.9 filed on Sep. 19, 2019 and Chinese Patent Application No. 202010424274.9 filed on May. 19, 2020, which are incorporated by reference in their entirety.

### TECHNICAL FIELD

The present invention relates to the field of antibody treatment, and in particular to use of an antibody specifically binding to proprotein convertase subtilisin/kexin type 9 (PCSK9) (referred to as anti-PCSK9 antibody hereinafter) and/or antibody fragment thereof and a pharmaceutical preparation comprising the same in a method for preventing or treating a cholesterol-related disease. Furthermore, the present invention relates to an anti-PCSK9 antibody or a liquid preparation thereof for preventing or treating the above disease.

### BACKGROUND

The incidence and mortality of coronary atherosclerotic heart disease and other atherosclerotic vascular diseases in China are on an increasing trend. Hypercholesterolemia is a known risk factor for coronary atherosclerotic heart disease, and increasing data suggests that active hypercholesterolemia treatment is related to a reduced risk of coronary atherosclerotic cardiovascular endpoint events. This relationship is particularly prominent in patients at high risk for cardiovascular events, such as patients with coronary heart disease.

At present, statins are cornerstones for cholesterol-lowering treatment, and play an important role in primary and secondary preventions of coronary atherosclerotic heart disease. However, current lipid-lowering treatment do not meet clinical demands. Although statins can reduce cardiovascular death, statin treatment has a certain limitation. First, statins reduce low-density lipoprotein cholesterol (LDL-C, LDL-cholesterol) by a maximum of 40-55%, and doubling the dose can reduce LDL-C by only about 6%. Furthermore, safety is a concern when using high-strength statins for Chinese population. More and more studies have shown that high-strength statin treatment is associated with higher myopathy and increased risk of liver enzymes, which is more prominent in Chinese population. HPS2-THRIVE study shows that when moderate-strength statin treatment is used, the incidence of adverse liver reactions of Chinese patients is obviously higher than that of European patients, the liver enzyme increasing rate (> 3 times of upper limit of normal value) of Chinese patients exceeds that of European patients by 10 times, and risk of myopathy of Chinese populations is also higher than that of European populations by 10 times (Zhao Shuiping, et al, Guidelines for the Prevention and Treatment of Dyslipidemia in Chinese Adults (2016 Revised Edition), China Circulation Journal 31(10):937-953, 2016). At present, there is no safety data about high-strength statin treatment for Chinese population.

Therefore, there is a need to continue to develop a new effective cholesterol-lowering treatment based on statins, which is of great clinical significance in reducing cardiovascular end-point events in high risk populations. At present, cholesterol-lowering drugs with multiple mechanisms of action have been launched or are under development. Among them, anti-PCSK-9 monoclonal antibodies have drawn extensive attention because of their good safety and efficacy (Zhao Shuiping, et al., Guidelines for the Prevention and Treatment of Dyslipidemia in Chinese Adults (2016 Revised Edition). Chinese Circulation Journal 31(10): 937-953, 2016). The PCSK-9 monoclonal antibody can inhibit PCSK-9, can block the binding of plasma PCSK-9 to low-density lipoprotein receptors (LDLRs), further prevent the endocytosis and degradation of LDLR, increase the expression level and number of LDLRs on cell surface, increase the reuptake of LDLR to LDL-C, finally reduce circulating LDL-C level, and achieve direct effect on lowering blood lipid level.

The PCSK-9 monoclonal antibody has significant curative effect, high safety and convenient subcutaneous injection. However, the PCSK-9 monoclonal antibody is administered once in 2 weeks or 4 weeks apart in the current administration cycle. Patient compliance is to be improved and there is an unmet clinical demand.

There remains a need for an improved method for treating or preventing a cholesterol-related disease which has a longer administration interval, thereby providing greater administration convenience to patients, and improving patient compliance; and/or the method has a fewer side effect or greater safety; and/or is easier to administer.

### SUMMARY

### I. Prevention or Treatment Method

In one aspect, the present invention relates to a method for lowering cholesterol level in a subject, which comprises administering to the subject an anti-PCSK9 antibody or antigen-binding fragment thereof at a dose of about 15 mg to 3500 mg.

In another aspect, the present invention relates to a method for preventing or treating a disorder related to increased LDL-cholesterol level in a subject, which comprises administering to the subject an anti-PCSK9 antibody or antigen-binding fragment thereof at a dose of about 15 mg to 3500 mg.

In one aspect, the present invention relates to a method for preventing or treating a cholesterol-related disease, such as hypercholesterolemia and/or hyperlipidemia, which comprises administering to the subject an anti-PCSK9 antibody or antigen-binding fragment thereof at a dose of about 15 mg to 3500 mg.

In some embodiments of the above methods, the anti-PCSK9 antibody or antigen-binding fragment thereof comprises a heavy chain variable region VH and a light chain variable region VL, wherein the VH comprises HCDR1, HCDR2 and HCDR3, wherein HCDR1 comprises or consists of an amino acid sequence set forth in SEQ ID NO: 10, HCDR2 comprises or consists of an amino acid sequence set forth in SEQ ID NO: 17, and HCDR3 comprises or consists of an amino acid sequence set forth in SEQ ID NO: 19; the VL comprises a combination of LCDR1, LCDR2, and LCDR3, wherein LCDR1 comprises or consists of an amino acid sequence set forth in SEQ ID NO: 4, LCDR2 comprises or consists of an amino acid sequence set forth in SEQ ID NO: 5, and LCDR3 comprises or consists of an amino acid sequence set forth in SEQ ID NO: 6.

In some embodiments of the above methods, the anti-PCSK9 antibody or antigen-binding fragment thereof comprises a heavy chain variable region VH and a light chain variable region VL, wherein the heavy chain variable region VH comprises or consists of an amino acid sequence set forth in SEQ ID NO: 30; the light chain variable region VL comprises or consists of an amino acid sequence set forth in SEQ ID NO: 24.

In some embodiments of the above methods, the anti-PCSK9 antibody or antigen-binding fragment thereof comprises a heavy chain and a light chain, wherein the heavy chain comprises or consists of an amino acid sequence set forth in SEQ ID NO: 41; the light chain comprises or consists of an amino acid sequence set forth in SEQ ID NO: 35.

In some embodiments of the above methods, the anti-PCSK9 antibody or antigen-binding fragment thereof is ADI-10087 disclosed in PCT patent application PCT/CN2017/118050.

In some embodiments of the above methods, the cholesterol is LDL-cholesterol.

In some embodiments of the above methods, the anti-PCSK9 antibody or antigen-binding fragment thereof is administered to the subject at a dose of about 25 mg to about 3000 mg, about 50 mg to about 2500 mg, about 75 mg to about 2000 mg, about 100 mg to about 2000 mg, about 200 mg to about 2000 mg, about 250 mg to about 1500 mg, about 300 mg to about 1000 mg, about 450 mg to about 1000 mg, about 600 mg to about 1000 mg, about 350 mg to about 900 mg, about 400 mg to about 800 mg, about 450 mg to about 700 mg, about 300 mg to about 600 mg, or about 450 mg to about 600 mg.

In some embodiments, the anti-PCSK9 antibody or antigen-binding fragment thereof is administered to a subject at a dose of about 25 mg, about 50 mg, about 75 mg, about 100 mg, about 125 mg, about 140 mg, about 150 mg, about 200 mg, about 250 mg, about 300 mg, about 350 mg, about 400 mg, about 420 mg, about 450 mg, about 500 mg, about 550 mg, about 600 mg, about 650 mg, about 700 mg, about 750 mg, about 800 mg, about 900 mg, about 1000 mg, about 1200 mg, about 1500 mg, about 2000 mg, about 2500 mg, about 3000 mg, or about 3500 mg.

In some embodiments of the above methods, the anti-PCSK9 antibody or antigen-binding fragment thereof is administered parenterally.

In some embodiments of the above methods, the anti-PCSK9 antibody or antigen-binding fragment thereof is administered subcutaneously.

In some embodiments of the above methods, the anti-PCSK9 antibody or antigen-binding fragment thereof is administered intravenously.

In some embodiments of the above methods, an LDL-cholesterol level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the LDL-cholesterol level in the subject before administration.

In some embodiments of the above methods, a serum free PCSK-9 level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the serum free PCSK-9 level in the subject before administration.

In some embodiments of the above methods, a total cholesterol level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the total cholesterol level in the subject before administration.

In some embodiments of the above methods, an apolipoprotein B level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the apolipoprotein B level in the subject before administration.

In some embodiments of the above methods, a non-high-density lipoprotein cholesterol level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the non-high-density lipoprotein cholesterol level in the subject before administration.

In some embodiments of the above methods, a lipoprotein a level in a subject is still decreased by > 20%, > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the lipoprotein a level in the subject before administration.

In some embodiments of the above methods, an apolipoprotein B/apolipoprotein A1 ratio level is still decreased by > 20%, > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the apolipoprotein B/apolipoprotein A1 ratio level in the subject before administration.

In some embodiments of the above methods, the cholesterol or the LDL-cholesterol is serum LDL-chole sterol.

In some embodiments of the above methods, the anti-PCSK9 antibody or antigen-binding fragment thereof is administered once at an interval equal to or greater than every four weeks (Q4W), e.g., once every five weeks (Q5W), once every six weeks (Q6W), once every seven weeks (Q7W), once every eight weeks (Q8W), once every nine weeks (Q9W), once every ten weeks (Q10W), once every ten weeks (Q11W), once every twelve weeks (Q12W), once every 4 months, once every 5 months, once every 6 months, once every 7 months, or once a year.

In some embodiments of the above methods, the subject, after administration, does not develop severe adverse events, particularly severe adverse events associated with the anti-PCSK9 antibody or antigen-binding fragment thereof.

In some embodiments of the above methods, the subject has an incidence of adverse events comparable to a subject receiving placebo after administration.

In some embodiments of the above methods, the subject is a human.

In some embodiments of the above methods, wherein the cholesterol-related disease is selected from homozygous familial hypercholesterolemia, heterozygous familial hypercholesterolemia and non-familial hypercholesterolemia.

In some embodiments of the above methods, the method comprises administering ADI-10087 subcutaneously or intravenously once every four weeks or six weeks at a dose of about 300 mg to 1000 mg (e.g., about 300 mg, about 350 mg, about 400 mg, about 420 mg, about 450 mg, about 500 mg, about 550 mg, about 600 mg, about 650 mg, about 700 mg, about 750 mg, about 800 mg, about 900 mg, or about 1000 mg) to a subject, and an LDL-cholesterol level in the subject is still decreased by > 50% four or six weeks after administration compared to the LDL-cholesterol level in the subject before administration.

In some embodiments of the above methods, the method comprises administering ADI-10087 subcutaneously or intravenously once every four weeks or six weeks at a dose of about 450 mg to 1000 mg (e.g., about 450 mg, about 500 mg, about 550 mg, about 600 mg, about 650 mg, about 700 mg, about 750 mg, about 800 mg, about 900 mg, or about 1000 mg) to a subject, and an LDL-cholesterol level in the subject is still decreased by > 50% four or six weeks after administration compared to the LDL-cholesterol level in the subject before administration.

In some embodiments of the above methods, the method comprises administering ADI-10087 subcutaneously or intravenously once every six weeks or eight weeks at a dose of about 600 mg to 1000 mg (e.g., about 600 mg, about 650 mg, about 700 mg, about 750 mg, about 800 mg, about 900 mg, or about 1000 mg) to a subject, and an LDL-cholesterol level in the subject is still decreased by > 50% six or eight weeks after administration compared to the LDL-cholesterol level in the subject before administration.

In some embodiments of the above methods, the method comprises administering ADI-10087 subcutaneously or intravenously once every four weeks or six weeks at a dose of about 300 mg to 1000 mg (e.g., about 300 mg, about 350 mg, about 400 mg, about 450 mg, about 500 mg, about 550 mg, about 600 mg, about 650 mg, about 700 mg, about 750 mg, about 800 mg, about 900 mg, or about 1000 mg) to a subject, and an LDL-cholesterol level in the subject is still decreased by > 50% four or six weeks after administration and a serum free PCSK-9 level in the subject is still decreased by > 60% four or six weeks after administration compared to those in the subject before administration.

In some embodiments of the above methods, the method comprises administering ADI-10087 subcutaneously or intravenously once every four weeks or six weeks at a dose of about 450 mg to 1000 mg (e.g., about 450 mg, about 500 mg, about 550 mg, about 600 mg, about 650 mg, about 700 mg, about 750 mg, about 800 mg, about 900 mg, or about 1000 mg) to a subject, and an LDL-cholesterol level in the subject is still decreased by > 50% four or six weeks after administration and a serum free PCSK-9 level in the subject is still decreased by > 80% four or six weeks after administration compared to those in the subject before administration.

In some embodiments of the above methods, the method comprises administering ADI-10087 subcutaneously or intravenously once every six weeks or eight weeks at a dose of about 600 mg to 1000 mg (e.g., about 600 mg, about 650 mg, about 700 mg, about 750 mg, about 800 mg, about 900 mg, or about 1000 mg) to a subject, and an LDL-cholesterol level in the subject is still decreased by > 50% six or eight weeks after administration and a serum free PCSK-9 level in the subject is still decreased by > 50% eight weeks after administration compared to those in the subject before administration.

In some specific embodiments of the above methods, the anti-PCSK9 antibody or antigen-binding fragment thereof is administered from a preparation described below.

In some specific embodiments of the above methods, the anti-PCSK9 antibody or antigen-binding fragment thereof is administered from a liquid antibody preparation, and the liquid antibody preparation comprises:
(i) about 100 mg/mL to about 200 mg/mL of the anti-PCSK-9 antibody or antigen-binding fragment thereof;
(ii) about 0.2 mg/mL to 10 mg/mL of histidine;
(iii) about 1% to 6% sorbitol; and
(iv) about 0.05 mg/mL to 1 mg/mL of polysorbate-80 or polysorbate-20;
wherein the pH of the liquid preparation is about 5.0 to 6.0.

In some specific embodiments of the above methods, the anti-PCSK9 antibody or antigen-binding fragment thereof is administered from a liquid antibody preparation, and the liquid antibody preparation comprises:
(i) about 100 mg/mL to about 200 mg/mL of the anti-PCSK-9 antibody or antigen-binding fragment thereof;
(ii) about 0.2 mg/mL to 10 mg/mL of histidine;
(iii) about 1% to 6% of sorbitol and about 50 mmol/L to 180 mmol/L of arginine or arginine salt; and
(iv) about 0.05 mg/mL to 1 mg/mL of polysorbate-80 or polysorbate-20;
wherein the pH of the liquid preparation is about 5.0 to 6.0.

In some specific embodiments of the above methods, the anti-PCSK9 antibody or antigen-binding fragment thereof is administered from a liquid antibody preparation, and the liquid antibody preparation uses water as a solvent and consists of the following compositions:

| | |
|---|---|
| Anti-PCSK9 antibody or antigen-binding fragment thereof | 150 mg/mL |
| Histidine | 1.5 g/L |
| Arginine | 90 mmol/L |
| Sorbitol | 3% (w/v) |
| Polysorbate 80 | 0.3 g/L |

wherein the pH of the liquid preparation is about 5.5.

In some specific embodiments, the liquid antibody preparation uses water as a solvent and consists of the following compositions:

| | |
|---|---|
| ADI-10087 | 150 mg/mL |
| Histidine | 1.5 g/L |
| Arginine | 90 mmol/L |
| Sorbitol | 3% (w/v) |
| Polysorbate 80 | 0.3 g/L |

wherein the pH of the liquid preparation is about 5.5.

It should be noted that the antibody or antigen-binding fragments thereof, sequence, preparation and biological activity and the like thereof used in the present invention have been disclosed in another PCT patent application PCT/CN2017/118050 (Publication No. WO2018/113781) of the present applicant, and the content of which is incorporated herein by reference in its entirety. For convenience of correspondence and reference, the sequences disclosed herein (including the sequence listing) and numbering thereof are directly cited from patent application PCT/CN2017/118050 without renumbering.

### II. Antibody Preparation Disclosed Herein

The methods of the present invention may use the antibody preparations described in 201810450088.5, which is incorporated by reference herein in its entirety. The anti-PCSK9 antibody preparation used in the present invention (hereinafter referred to as "the antibody preparation disclosed herein") is also described in detail below.

In some specific embodiments of the above methods, the anti-PCSK9 antibody or antigen-binding fragment thereof is administered from a preparation described below.

In one aspect, the present invention may use the following liquid antibody preparation (hereinafter referred to as "the liquid antibody preparation disclosed herein"), which comprises:
(i) an anti-PCSK-9 antibody or a fragment thereof (such as an antigen-binding fragment) as defined herein;
(ii) a buffer;
(iii) a viscosity inhibitor; and
(iv) a surfactant.

In some embodiments, the anti-PCSK9 antibody or antigen-binding fragment thereof comprises a heavy chain variable region VH and a light chain variable region VL, wherein the VH comprises three complementarity determining regions (CDRs) contained in a VH set forth in SEQ ID NO: 30; the VL comprises three complementarity determining regions (CDRs) contained in a VL set forth in SEQ ID NO: 24.

In some embodiments of the above methods, the anti-PCSK9 antibody or antigen-binding fragment thereof comprises a heavy chain variable region VH and a light chain variable region VL, wherein the VH comprises a combination of HCDR1, HCDR2 and HCDR3, wherein HCDR1 comprises or consists of an amino acid sequence set forth in SEQ ID NO: 10, HCDR2 comprises or consists of an amino acid sequence set forth in SEQ ID NO: 17, and HCDR3 comprises or consists of an amino acid sequence set forth in SEQ ID NO: 19; the VL comprises a combination of LCDR1, LCDR2 and LCDR3 comprising or consisting of amino acid sequences set forth in SEQ ID NOs: 4, 5 and 6, respectively.

In some embodiments of the above methods, the anti-PCSK9 antibody or antigen-binding fragment thereof comprises a heavy chain variable region VH and a light chain variable region VL, wherein the heavy chain variable region VH comprises or consists of an amino acid sequence of SEQ ID NO: 30; the light chain variable region VL comprises or consists of an amino acid sequence of SEQ ID NO: 24.

In some embodiments of the above methods, the anti-PCSK9 antibody or antigen-binding fragment thereof comprises a heavy chain and a light chain, wherein the heavy chain comprises or consists of an amino acid sequence of SEQ ID NO: 41; the light chain comprises or consists of an amino acid sequence of SEQ ID NO: 35.

In some embodiments of the above methods, the anti-PCSK9 antibody is ADI-10087 disclosed in Chinese patent application PCT/CN2017/118050.

In one embodiment, the concentration of the anti-PCSK9 antibody or antigen-binding fragment thereof in the liquid antibody preparation disclosed herein is about 50 mg/mL to about 200 mg/mL. In another embodiment, the concentration of the anti-PCSK9 antibody or antigen-binding fragment thereof in the liquid antibody preparation disclosed herein is about 100 mg/mL to about 200 mg/mL. In another embodiment, the concentration of the anti-PCSK9 antibody or antigen-binding fragment thereof in the liquid antibody preparation disclosed herein is about 100 mg/mL. In another embodiment, the concentration of the anti-PCSK9 antibody or antigen-binding fragment thereof in the liquid antibody preparation disclosed herein is about 125 mg/mL. In another embodiment, the concentration of the anti-PCSK9 antibody or antigen-binding fragment thereof in the liquid antibody preparation disclosed herein is about 150 mg/mL. In another embodiment, the concentration of the anti-PCSK9 antibody or antigen-binding fragment thereof in the liquid antibody preparation disclosed herein is about 175 mg/mL. In another embodiment, the concentration of the anti-PCSK9 antibody or antigen-binding fragment thereof in the liquid antibody preparation disclosed herein is about 200 mg/mL.

In one embodiment, the anti-PCSK9 antibody is any antibody binding to PCSK9 protein (such as human PCSK9), such as a polyclonal antibody, a monoclonal antibody or a combination of the two. Preferably, in one embodiment, the anti-PCSK9 antibody is a monoclonal antibody. In one embodiment, the anti-PCSK9 antibody or antigen-binding fragment thereof is the anti-PCSK9 antibody or antigen-binding fragment thereof defined herein.

In one embodiment, the concentration of the buffer in the liquid antibody preparation disclosed herein is about 0.01 mg/mL to 50 mg/mL. In one embodiment, the concentration of the buffer in the liquid antibody preparation disclosed herein is about 0.1 mg/mL to 50 mg/mL. In one embodiment, the concentration of the buffer in the liquid antibody preparation disclosed herein is about 0.2 mg/mL to 10 mg/mL. In one embodiment, the concentration of the buffer in the liquid antibody preparation disclosed herein is about 0.5 mg/mL to 2.5 mg/mL. In one embodiment, the concentration of the buffer in the liquid antibody preparation disclosed herein is about 1.0 mg/mL to 2.0 mg/mL. In one embodiment, the concentration of the buffer in the liquid antibody preparation disclosed herein is about 1.5 mg/mL.

In one embodiment, the buffer is selected from histidine, glutamate, phosphate, acetate, citrate, and tris(hydroxymethyl)aminomethane. In one embodiment, the buffer is histidine.

In one embodiment, the concentration of the viscosity inhibitor in the liquid antibody preparation disclosed herein is about 10 mmol/L to 1000 mmol/L. In one embodiment, the concentration of the viscosity inhibitor in the liquid antibody preparation disclosed herein is about 20 mmol/L to 500 mmol/L. In one embodiment, the concentration of the viscosity inhibitor in the liquid antibody preparation disclosed herein is about 50 mmol/L to 300 mmol/L. In one embodiment, the concentration of the viscosity inhibitor in the liquid antibody preparation disclosed herein is about 50 mmol/L to 200 mmol/L.

In one embodiment, the viscosity inhibitor is selected from sugar alcohols (such as sorbitol), arginine, arginine hydrochloride, sodium thiocyanate, ammonium thiocyanate, ammonium sulfate, ammonium chloride, calcium chloride, zinc chloride, sodium acetate, and combinations thereof. In one embodiment, the viscosity inhibitor is sorbitol. In one embodiment, the viscosity inhibitor is sorbitol with a concentration of about 1% to 6% (w/v). In one embodiment, the viscosity inhibitor is sorbitol with a concentration of about 1% to 6% (w/v), and the liquid preparation does not comprise other viscosity inhibitors. In one embodiment, the viscosity inhibitor is a combination of sorbitol and arginine or arginine salt (preferably arginine hydrochloride). In one embodiment, the viscosity inhibitor is a combination of sorbitol and arginine or arginine salt (preferably arginine hydrochloride), wherein the concentration of sorbitol is about 1% to 6% (w/v), preferably about 2% to 4% (w/v), and more preferably about 3% (w/v), and the concentration of arginine or arginine salt (preferably arginine hydrochloride) is about 50 mmol/L to 180 mmol/L, preferably about 70 mmol/L to 150 mmol/L, and more preferably about 90 mmol/L. In one embodiment, the viscosity inhibitor is a combination of sorbitol and arginine or arginine salt (preferably arginine hydrochloride), wherein the concentration of sorbitol is about 1% to 6% (w/v), preferably about 2% to 4% (w/v), and more preferably about 3% (w/v), and the concentration of arginine or arginine salt is about 50 mmol/L to 180 mmol/L, preferably about 70 mmol/L to 150 mmol/L, and more preferably about 90 mmol/L, and the liquid preparation does not comprise other viscosity inhibitors.

In one embodiment, the concentration of the surfactant is about 0.01 mg/mL to 5 mg/mL. In one embodiment, the concentration of the surfactant is about 0.05 mg/mL to 1 mg/mL. In one embodiment, the concentration of the surfactant is about 0.1 mg/mL to 0.5 mg/mL. In one embodiment, the concentration of the surfactant is about 0.3 mg/mL.

In one embodiment, the surfactant is a nonionic surfactant. In one embodiment, the surfactant is, for example, pluronics, polysorbate-80, polysorbate-60, polysorbate-40, or polysorbate-20.

In one embodiment, the liquid preparation disclosed herein comprises a solvent, and preferably, the solvent is selected from water for injection, organic solvents for injection (including but not limited to oil for injection, ethanol, and propylene glycol, and the like), and combinations thereof.

In one embodiment, the pH of the liquid preparation is about 5.0 to 6.0. In one embodiment, the pH of the liquid preparation is about 5.2 to 5.8. In one embodiment, the pH of the liquid preparation is about 5.4 to 5.6. In one embodiment, the pH of the liquid preparation is about 5.5.

In one embodiment, the viscosity of the liquid preparation at 25 °C is about 1.0 centipoises to 20 centipoises.In one embodiment, the viscosity of the liquid preparation at 25 °C is about 1.0 centipoises to 10 centipoises. In one embodiment, the viscosity of the liquid preparation at 25 °C is about 1.0 centipoise, 2.0 centipoises, 3.0 centipoises, 4.0 centipoises, 5.0 centipoises, 6.0 centipoises, 7.0 centipoises, 8.0 centipoises, 9.0 centipoises, or 10.0 centipoises. In one embodiment, the viscosity of the liquid preparation at 25 °C is about 5.0 centipoises to 7.0 centipoises. In one embodiment, the viscosity of the liquid preparation at 25 °C is about 5.2 centipoises. In one embodiment, the viscosity of the liquid preparation at 25 °C is about 6.0 centipoises.

In one embodiment, the liquid preparation is a pharmaceutical preparation, preferably an injection, more preferably a subcutaneous injection.

In one preferred embodiment, the liquid antibody preparation disclosed herein comprises:
(i) about 100 mg/mL to about 200 mg/mL of the anti-PCSK-9 antibody or antigen-binding fragment thereof;
(ii) about 0.2 mg/mL to 10 mg/mL of histidine;
(iii) about 1% to 6% (w/v) of sorbitol; and
(iv) about 0.05 mg/mL to 1 mg/mL of polysorbate-80 or polysorbate-20;
wherein the pH of the liquid preparation is about 5.0 to 6.0.

In one more preferred embodiment, the liquid antibody preparation disclosed herein also comprises water for injection.

In one preferred embodiment, the liquid antibody preparation disclosed herein comprises:
(i) about 100 mg/mL to about 200 mg/mL of the anti-PCSK-9 antibody or antigen-binding fragment thereof;
(ii) about 0.2 mg/mL to 10 mg/mL of histidine;
(iii) about 1% to 6% of sorbitol and about 50 mmol/L to 180 mmol/L of arginine or arginine salt; and
(iv) about 0.05 mg/mL to 1 mg/mL of polysorbate-80 or polysorbate-20;
wherein the pH of the liquid preparation is about 5.0 to 6.0.

In one more preferred embodiment, the liquid antibody preparation disclosed herein also comprises water for injection.

In some specific embodiments, the liquid antibody preparation uses water as a solvent and consists of the following compositions:

| | |
|---|---|
| Anti-PCSK9 antibody or antigen-binding fragment thereof | 150 mg/mL |
| Histidine | 1.5 g/L |
| Arginine | 90 mmol/L |
| Sorbitol | 3% (w/v) |
| Polysorbate 80 | 0.3 g/L |

wherein the pH of the liquid preparation is about 5.5.

In some specific embodiments, the liquid antibody preparation uses water as a solvent and consists of the following compositions:

| | |
|---|---|
| ADI-10087 | 150 mg/mL |
| Histidine | 1.5 g/L |
| Arginine | 90 mmol/L |
| Sorbitol | 3% (w/v) |
| Polysorbate 80 | 0.3 g/L |

wherein the pH of the liquid preparation is about 5.5.

In one more preferred embodiment, the liquid antibody preparation disclosed herein also comprises water for injection.

In one embodiment, after the liquid preparation comprising an anti-PCSK9 antibody disclosed herein is stored at about -80 °C to about 45 °C, such as -80 °C, about -30 °C, about -20 °C, about 0 °C, about 5 °C, about 25 °C, about 35 °C, about 37 °C, about 42 °C or about 45 °C, for at least 14 days, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer, the purity of the anti-PCSK-9 antibody or antigen-binding fragment thereof decreases by no more than 10%, for example, no more than 5%, 4%, 3%, 2%, 1%, 0.5%, or 0.1%, as detected by SEC-HPLC and non-reduced CE-SDS.

In one embodiment, after the liquid preparation comprising an anti-PCSK9 antibody disclosed herein is stored at about -80 °C to about 45 °C, such as -80 °C, about -30 °C, about -20 °C, about 0 °C, about 5 °C, about 25 °C, about 35 °C, about 37 °C, about 42 °C or about 45 °C, for at least 14 days, at least 28 days, at least 1 month, at least 2 months, at least 3 months, at least 4 months, at least 5 months, at least 6 months, at least 7 months, at least 8 months, at least 9 months, at least 10 months, at least 11 months, at least 12 months, at least 18 months, at least 24 months, at least 36 months, or longer, the charge heterogeneity of the anti-PCSK-9 antibody or antigen-binding fragment thereof changes by no more than 10%, for example, no more than 5%, 4%, 3%, 2%, 1%, 0.5%, or 0.1%, as detected by CEX-HPLC.

The preparation disclosed herein has a high antibody concentration, a low viscosity suitable for administration (particularly subcutaneous administration), and a high physical and chemical stability, and is less prone to aggregate and granulate and exhibit charge heterogeneity change when stored. These advantages are very beneficial to the production of complete, effective, and highly consistent clinical drugs.

The inventors surprisingly found that sorbitol can be used as a viscosity inhibitor for the liquid preparation comprising an anti-PCSK9 antibody, in which case a liquid preparation comprising an anti-PCSK-9 antibody or antigen-binding fragment thereof having low viscosity and high concentration can be obtained without adding other viscosity inhibitors, and the liquid preparation has high stability. In addition, the inventors surprisingly found that a combination of sorbitol and arginine as a viscosity inhibitor can better reduce the viscosity of the liquid preparation comprising an anti-PCSK9 antibody and enable a high-concentration liquid preparation comprising an anti-PCSK9 antibody or antigen-binding fragment thereof to have lower viscosity suitable for subcutaneous injection lower viscosity suitable for subcutaneous injection, and the liquid preparation comprising an anti-PCSK9 antibody has higher stability.

In another aspect, the present invention provides a solid preparation obtained by lyophilizing the aforementioned liquid preparation. Before use, the solid preparation can be reconstituted in a suitable solvent to give the liquid preparation disclosed herein.

### III. Anti-PCSK9 Antibody or Preparation Thereof for Treating or Preventing Diseases

The present invention relates to an anti-PCSK9 antibody or antigen-binding fragment thereof or liquid antibody preparation thereof as described above for use in the methods of the present invention described above.

It will be appreciated that the dose administered refers to the amount of anti-PCSK9 antibody in the preparation when in the above embodiments relating to liquid preparations.

### IV. Single Pharmaceutical Dosage Unit, Pharmaceutical Kit, or Pharmaceutical Use Thereof

In another aspect, the present invention relates to a single pharmaceutical dosage unit, which comprises an anti-PCSK9 antibody or antigen-binding fragment thereof at a dose of about 15 mg to 3500 mg; preferably, an anti-PCSK9 antibody or antigen-binding fragment thereof at a dose of about 25 mg to about 3000 mg, about 50 mg to about 2500 mg, about 75 mg to about 2000 mg, about 100 mg to about 2000 mg, about 200 mg to about 2000 mg, about 250 mg to about 1500 mg, about 300 mg to about 1000 mg, about 450 mg to about 1000 mg, about 600 mg to about 1000 mg, about 350 mg to about 900 mg, about 400 mg to about 800 mg, about 450 mg to about 700 mg, about 300 mg to about 600 mg, or about 450 mg to about 600 mg.

In some embodiments, the anti-PCSK9 antibody or antigen-binding fragment thereof comprises a heavy chain variable region VH and a light chain variable region VL, wherein
(a) the VH comprises:
   (i) a combination of HCDR1, HCDR2 and HCDR3, wherein HCDR1 comprises or consists of an amino acid sequence set forth in SEQ ID NO: 10; HCDR2 comprises or consists of an amino acid sequence set forth in SEQ ID NO: 17; HCDR3 comprises or consists of an amino acid sequence set forth in SEQ ID NO: 19; or
   (ii) three complementarity determining regions (CDRs) contained in a VH set forth in SEQ ID NO: 30; and
(b) the VL comprises:
   (i) a combination of LCDR1, LCDR2 and LCDR3, wherein LCDR1 comprises or consists of an amino acid sequence set forth in SEQ ID NO: 4; LCDR2 comprises or consists of an amino acid sequence set forth in SEQ ID NO: 5; LCDR3 comprises or consists of an amino acid sequence set forth in SEQ ID NO: 6; or
   (ii) three complementarity determining regions (CDRs) contained in a VL set forth in SEQ ID NO: 24.

In some embodiments, the anti-PCSK9 antibody or antigen-binding fragment thereof comprises a heavy chain variable region VH and a light chain variable region VL, wherein,
(a) the heavy chain variable region VH comprises or consists of an amino acid sequence set forth in SEQ ID NO: 30; and
(b) the light chain variable region VL comprises or consists of an amino acid sequence set forth in SEQ ID NO: 24.

In some embodiments, the anti-PCSK9 antibody or antigen-binding fragment thereof comprises a heavy chain and a light chain, wherein
(a) the heavy chain comprises or consists of an amino acid sequence set forth in SEQ ID NO: 41; and
(b) the light chain comprises or consists of an amino acid sequence set forth in SEQ ID NO: 35; preferably, the anti-PCSK9 antibody is ADI-10087.

In another aspect, the present invention relates to a pharmaceutical kit, which comprises the anti-PCSK9 antibody or antigen-binding fragment thereof according to any one of the above embodiments of the single pharmaceutical dosage unit.

In another aspect, the present invention relates to use of the single pharmaceutical dosage unit as described above or the kit of parts as described above in preparing a medicament for lowering cholesterol level in a subject.

In another aspect, the present invention relates to use of the single pharmaceutical dosage unit as described above or the kit of parts as described above in preparing a medicament for the prevention or treatment of a disorder related to increased LDL-cholesterol level in a subject.

In another aspect, the present invention relates to use of the single pharmaceutical dosage unit as described above or the kit of parts as described above in preparing a medicament for the prevention or treatment of a cholesterol-related disease such as hypercholesterolemia and/or hyperlipidemia.

It should be understood that technical solutions obtained by combining the technical features described in section I and section III above and the technical solutions in section IV above are also included in the present application.

### BRIEF DESCRIPTON OF THE DRAWINGS:

FIG. 1 shows average free concentration-time curves of ADI-10087 after administration of ADI-10087 to subjects in each dose group; the upper graph is a linear coordinate; the bottom graph is a semi-logarithmic coordinate; error bars are represented as mean ± standard deviation, and each dose group is represented as a line.
FIG. 2 shows average total concentration-time curves of ADI-10087 after administration of ADI-10087 to subjects in each dose group; the upper graph is a linear coordinate; the bottom graph is a semi-logarithmic coordinate; error bars are represented as mean ± standard deviation, and each dose group is represented as a line.
FIG. 3 shows Cₘₐₓ distribution of total ADI-10087 and free ADI-10087 after abdominal subcutaneous injection of 25-600 mg of ADI-10087 to subjects; (scatter plot) the fold line in the plot is median value of each dose group; the scatter point is the individual value; the solid line is the corresponding parameter for free ADI-10087; the dashed line is the corresponding parameter for total ADI-10087.
FIG. 4 shows AUC₀-ₗₐₛₜ distribution of total ADI-10087 and free ADI-10087 after abdominal subcutaneous injection of 25-600 mg of ADI-10087 to subjects; (scatter plot) the fold line in the plot is median value of each dose group; the scatter point is the individual value; the solid line is the corresponding parameter for free ADI-10087; the dashed line is the corresponding parameter for total ADI-10087.
FIG. 5 shows AUC₀-_{∞} distribution of total ADI-10087 and free ADI-10087 after abdominal subcutaneous injection of 25-600 mg of ADI-10087 to subjects; (scatter plot) the fold line in the plot is median value of each dose group; the scatter point is the individual value; the solid line is the corresponding parameter for free ADI-10087; the dashed line is the corresponding parameter for total ADI-10087.
FIG. 6 shows change rate (%) of serum free PCSK-9 concentration relative to baseline after a single administration.
FIG. 7 shows a line graph of the least squares mean of LDL-C changes relative to baseline after a single administration.
FIG. 8 shows average change rate of LDL-C concentration relative to baseline after a single administration.
FIG. 9 shows average concentration-time curves of LDL-C after a single administration.
FIG. 10 shows change rate of total cholesterol concentration relative to baseline after a single administration.
FIG. 11 shows change rate of ApoB concentration relative to baseline after a single administration.
FIG. 12 shows change rate of Non-HDL-C concentration relative to baseline after a single administration.
FIG. 13 shows change rate of lipoprotein a (Lp(a)) concentration relative to baseline after a single administration.
FIG. 14 shows change rate (%) of serum free PCSK-9 concentration relative to baseline after multiple administrations.
FIG. 15 shows change rate of an LDL-C level relative to baseline after multiple administrations.
FIG. 16 shows changes in an LDL-C level relative to baseline after multiple administrations.
FIG. 17 shows change rate of an Lp(a) level relative to baseline after multiple administrations.
FIG. 18 shows change rate of an ApoB level relative to baseline after multiple administrations.
FIG. 19 shows change rate of a non-HDL-C level change relative to baseline after multiple administrations.
FIG. 20 shows change rate of a total cholesterol TC level relative to baseline after multiple administrations.
FIG. 21 shows change rate of an ApoB/ApoAl level relative to baseline after multiple administrations.

### DETAILED DESCRIPTION

### Definitions

The terms used in the present invention have the following definitions. If not defined herein, the definitions in patent application PCT/CN2017/118050 are preferably applied. If no definitions are given in both patent applications, the terms used in the present invention have the meanings commonly understood in the art.

For the purpose of explaining this specification, the following definitions will be used, and wherever appropriate, terms used in the singular form may also include the plural form, and vice versa. It should be understood that the terms used herein are for the purpose of describing specific embodiments only, and are not intended to be limiting.

As used herein, the term "and/or" refers to any one of the options or two or more of the options.

As used herein, the term "comprise" or "include" is intended to mean that the described elements, integers or steps are included, but not to the exclusion of any other elements, integers or steps. The term "comprise" or "include" used herein, unless otherwise specified, also encompasses the situation where the entirety consists of the described elements, integers or steps. For example, when referring to an antibody variable region "comprising" a particular sequence, it is also intended to encompass an antibody variable region consisting of the particular sequence.

"Antibodies or antigen-binding fragments thereof" applicable to the present invention include but are not limited to polyclonal, monoclonal, monovalent, bispecific, isoconjugate, multispecific, recombinant, heterogenous, heterogenous hybrid, chimeric, humanized (particularly CDR-grafted), deimmunized or human antibodies, Fab fragments, Fab' fragments, F(ab')₂ fragments, fragments produced by an Fab expression library, Fd, Fv, disulphide-linked Fv (dsFv), single-chain antibodies (such as scFv), diabodies or tetrabodies (Holliger P. et al. (1993) Proc.Natl.Acad.Sci.U.S.A.90(14), 6444-6448), nanobodies (also referred to as single-domain antibodies), anti-idiotype (anti-Id) antibodies (including, for example, anti-Id antibodies against the antibody disclosed herein), and an epitope-binding fragment of any of the above.

"Individual" or "subject" includes mammals. The mammals include, but are not limited to, domestic animals (e.g., cattle, goats, cats, dogs, and horses), primates (e.g., human and non-human primates such as monkeys), rabbits, and rodents (e.g., mice and rats). In some embodiments, the individual or subject is a human, including a child, a teenager or an adult.

The term "combination therapy" means that two or more therapeutic agents are administered to treat cholesterol-related diseases as described herein. Such administration includes co-administration of these therapeutic agents in a substantially simultaneous manner, for example, in a single capsule with a fixed proportion of active ingredients. Alternatively, such administration includes co-administration of each active ingredient in a variety of or separate containers (such as tablets, capsules, powder and liquid). The powder and/or liquid can be reconstituted or diluted to a desired dosage before administration. In addition, such administration also includes using each type of therapeutic agents in a sequential manner at approximately the same time or at different times. In any case, the therapeutic regimen will provide the beneficial effect of the drug combination in the treatment of disorders or symptoms described herein.

As used herein, "treatment" (or "treat" or "treating") refers to slowing, interrupting, arresting, alleviating, stopping, lowering, or reversing the progression or severity of an existing symptom, disorder, condition, or disease.

As used herein, "prevention" (or "prevent" or "preventing") includes the inhibition of the onset or progression of symptoms of a disease or disorder, or a specific disease or disorder. In some embodiments, subjects with family histories are candidates for prophylactic regimens. Generally, the term "prevention" (or "prevent" or "preventing") refers to the administration of a drug prior to the onset of disorders or symptoms, particularly in subjects at risk.

The term "cholesterol-related disease" includes any one or more of the following: hypercholesterolemia, hyperlipidaemia, heart disease, metabolic syndrome, diabetes mellitus, coronary heart disease, stroke, cardiovascular diseases, Alzheimers disease, and general dyslipidemia (shown as, for example, increased total serum cholesterol, increased LDL, increased triglyceride, increased VLDL, and/or low HDL). Some non-limiting examples of primary and secondary dyslipidemias that can be treated with an anti-PCSK9 antibody (alone or in combination with one or more other drugs) include metabolic syndrome, diabetes mellitus, familial combined hyperlipidemia, familial hypertriglyceridemia, familial hypercholesterolemias, including heterozygous hypercholesterolemia, homozygous hypercholesterolemia, and familial defective apolipoprotein B-100; polygenic hypercholesterolemia; homozygous familial hypercholesterolemia, heterozygous familial hypercholesterolemia, and non-familial hypercholesterolemia; remnant removal disease; hepatic lipase deficiency; dyslipidemia secondary to any of the following: dietary indiscretion, hypothyroidism, drugs (including estrogen and progesterone therapies, β blockers and thiazide diuretics); nephrotic syndrome, chronic renal failure, Cushing's syndrome, primary biliary cirrhosis, glycogen storage diseases, hepatoma, cholestasis, acromegaly, insulinoma, isolated growth hormone deficiency, and alcohol-induced hypertriglyceridemia.

The term "hypercholesterolemia" used herein refers to a disorder in which the cholesterol level rises to above a certain level. In some embodiments, the LDL-cholesterol level rises to above a certain level. In some embodiments, the serum LDL-cholesterol level rises to above a certain level.

The term "vector" used herein refers to a nucleic acid molecule capable of proliferating another nucleic acid to which it is linked. The term includes vectors that serve as self-replicating nucleic acid structures as well as vectors binding to the genome of a host cell into which they have been introduced. Some vectors are capable of directing the expression of a nucleic acid to which they are operably linked. Such vectors are called "expression vectors" herein.

The term "effective amount" refers to an amount or a dosage of the preparation, antibody, or fragment described herein which generates an expected effect in a treated patient after the administration of a single or multiple doses. The effective amount can be easily determined by an attending physician as a person skilled in the art by considering a variety of factors as follows: species such as mammals; its size, age, and general health condition; the specific disease involved; the extent or severity of the disease; response in an individual patient; specific antibody administered; route of administration; bioavailability characteristics of the administered preparation; selected administration regimen; and use of any concomitant therapy.

The "therapeutically effective amount" refers to an amount effective to achieve a desired therapeutic result at a necessary dosage for a necessary period of time. The therapeutically effective amount of the preparation, antibody or antibody fragment described herein, or conjugate or composition thereof can vary depending on a variety of factors such as disease state, age, sex and weight of an individual, and the ability of the antibody or antibody portion to elicit a desired response in the individual. The therapeutically effective amount is also such an amount in which any toxic or undesired effect of the preparation, antibody or antibody fragment or conjugate or composition thereof is inferior to the therapeutically beneficial effect.

The "prophylactically effective amount" refers to an amount effective to achieve a desired prophylactic result at a necessary dosage for a necessary period of time. Generally, since a prophylactic dose is administered in a subject before or at an earlier stage of a disease, a prophylactically effective amount will be less than a therapeutically effective amount.

"Single pharmaceutical dosage unit" represents a single pharmaceutical dosage form including injections, tablets, and lyophilized powders, administered to a patient at the time of an administration regimen.

As used herein, the term "preparation" refers to a composition which is suitably administered to animals, preferably mammals (including humans) and comprises at least one active ingredient and at least one non-active ingredient. "Liquid preparation" refers to a preparation in the form of liquid. The liquid preparation disclosed herein comprises (i) an anti-PCSK-9 antibody or fragment thereof (preferably an antigen-binding fragment); (ii) a buffer; (iii) a viscosity inhibitor; (iv) a surfactant; and (v) a solvent. The composition of the preparation disclosed herein may be as shown in the aforementioned liquid preparation embodiments. The liquid preparation disclosed herein is preferably an injection, more preferably a subcutaneous injection.

As used herein, the "buffer" refers to a pH buffer. Preferably, the buffer can keep pH of the liquid preparation disclosed herein at about 5.0 to 6.0, preferably about 5.2 to 5.8, more preferably about 5.4 to 5.6, and most preferably about 5.5. The concentration of the buffer in the liquid preparation is, for example, about 0.01 mg/mL to 50 mg/mL, about 0.1 mg/mL to 50 mg/mL, about 0.2 mg/mL to 10 mg/mL, about 0.5 mg/mL to 2.5 mg/mL, about 1.0 mg/mL to 2.0 mg/mL, or about 1.5 mg/mL. Preferably, the buffer is selected from histidine, glutamate, phosphate, acetate, citrate and tris(hydroxymethy l )aminomethane.

As used herein, the "viscosity inhibitor" refers to a substance that can reduce the viscosity of the liquid preparation comprising an anti-PCSK9 antibody. The concentration of the viscosity inhibitor in the liquid preparation is, for example, about 10 mmol/L to 1000 mmol/L, about 20 mmol/L to 500 mmol/L, about 50 mmol/L to 300 mmol/L, or about 50 mmol/L to 200 mmol/L. Preferably, the viscosity inhibitor is selected from sugar alcohol, arginine, arginine hydrochloride, sodium thiocyanate, ammonium thiocyanate, ammonium sulfate, ammonium chloride, calcium chloride, zinc chloride, sodium acetate, and combinations thereof. More preferably, the viscosity inhibitor is sorbitol or a combination of sorbitol and arginine or arginine salt (preferably arginine hydrochloride). Preferably, the concentration of sorbitol in the liquid preparation is about 1% to 6% (w/v); and the concentration of arginine or arginine salt (preferably arginine hydrochloride) in the liquid preparation is about 50 mmol/L to 180 mmol/L, preferably about 70 mmol/L to 150 mmol/L, and more preferably about 90 mmol/L.

As described herein, the "surfactant" refers to a substance which can significantly change the interface state of a solution system after being added in a small amount. The concentration of the surfactant in the liquid preparation is, for example, about 0.01 mg/mL to 5 mg/mL, about 0.05 mg/mL to 1 mg/mL, or about 0.1 mg/mL to 0.5 mg/mL, and more preferably about 0.3 mg/mL. Preferably, the surfactant is a nonionic surfactant, such as pluronics, polysorbate-80, polysorbate-60, polysorbate-40, or polysorbate-20.

As used herein, the term "solvent" refers to a liquid that is used to dissolve or suspend active ingredients and non-active ingredients to form a liquid preparation. Solvents that can be used in the present invention include, but are not limited to, water for injection, organic solvents for injection (including but not limited to oil for injection, ethanol, and propylene glycol), and combinations thereof.

As used herein, the term "sugar alcohol" refers to a corresponding polyol which is obtained by reduction reaction, such as catalytic hydrogenation, of monosaccharide. Sugar alcohols include, but are not limited to, sorbitol, mannitol, erythritol, maltitol, lactitol, xylitol, etc.

The term "about" used in combination with a numerical value is intended to encompass the numerical values in a range from a lower limit less than the specified numerical value by 10% to an upper limit greater than the specified numerical value by 10%.

As used herein, "w/v" refers to "weight/volume", for example, "1% w/v" means 1 g/100 mL = 0.01 g/mL = 10 mg/mL.

"Administering" and "administration" are used interchangeably herein, unless otherwise specifically indicated.

A pharmaceutical kit can be also referred to as a kit of parts herein.

The present invention will be further illustrated below with reference to examples, and the following examples should not be construed as limiting the present invention.

Abbreviations:
SEC-HPLC: size exclusion chromatography-high performance liquid chromatography
CE-SDS: capillary electrophoresis-sodium dodecyl sulfate
CZE: capillary zone electrophoresis
SC: subcutaneous injection
IV: intravenous injection
PK: pharmacokinetics
LDL-C: low-density lipoprotein cholesterol

### Example 1. Preparation of the Liquid Preparation Comprising Anti-PCSK9 Antibody (Preparation A, i.e., Test Drug)

A liquid preparation comprising an anti-PCSK9 antibody with a concentration of 150 mg/mL prepared according to formula A

### Formula A

| | |
|---|---|
| Anti-PCSK9 antibody | 150 mg/mL |
| (ADI-10087) | |
| Histidine | 1.5 g/L |
| Arginine | 90 mmol/L |
| Sorbitol | 3% (w/v) |
| Polysorbate 80 | 0.3 g/L |
| pH | 5.5 |

The anti-PCSK9 antibody (ADI-10087) obtained from patent application PCT/CN2017/118050 was added to an ultrafiltration centrifuge tube (molecular weight cutoff: 30 KD), after concentration by centrifugation, the anti-PCSK9 antibody was diluted with an aqueous solution of formula A without polysorbate 80 and the anti-PCSK9 antibody (1.5 g/L histidine, 90 mmol/L arginine, 3% sorbitol, pH 5.5) and then concentrated; and this operation was repeated until the substitution was completed. The concentration of the substituted protein was adjusted to 150 mg/mL. Then an aqueous solution of polysorbate 80 (30 g/L, 1/100 by volume) was added to adjust the final concentration of polysorbate 80 to 0.3 g/L. After being aseptically filtered, the semi-finished product was aliquoted into vials and the vials were then capped with rubber stoppers and aluminum-plastic caps, and the product was obtained.

### Example 2. Preparation of Comparative Example (Preparation B) of the Liquid Preparation Comprising Anti-PCSK9 Antibody

Comparative example: A preparation comprising anti-PCSK9 antibody liquid with a concentration of 150 mg/mL prepared according to formula B

### Formula B

| | |
|---|---|
| Anti-PCSK9 antibody | 150 mg/mL |
| (ADI-10087) | |
| Histidine | 1.5 g/L |
| Arginine | 180 mmol/L |
| Polysorbate 80 | 0.3 g/L |
| pH | 5.5 |

The anti-PCSK9 antibody (ADI-10087) obtained from patent application PCT/CN2017/118050 was added to an ultrafiltration centrifuge tube (molecular weight cutoff: 30 KD), after concentration by centrifugation, the anti-PCSK9 antibody was diluted with an aqueous solution of formula B without polysorbate 80 and the anti-PCSK9 antibody (1.5 g/L histidine, 180 mmol/L arginine, pH 5.5) and then concentrated; and this operation was repeated until the substitution was completed. The concentration of the substituted protein was adjusted to 150 mg/mL. Then an aqueous solution of polysorbate 80 (30 g/L, 1/100 by volume) was added to adjust the final concentration of polysorbate 80 to 0.3 g/L. After being aseptically filtered, the semi-finished product was aliquoted into vials and the vials were then capped with rubber stoppers and aluminum-plastic caps, and the product was obtained.

### Example 3. Measurement of Viscosity of the Liquid Preparation Comprising Anti-PCSK9 Antibody

The viscosity was measured with a cone-and-plate viscometer (Brookfield, CAP1000+/2000+. For details, visit http://www.sinoinstrument.com/product_details-4-83-401-60.html) at room temperature of 20 °C to 25 °C. A rotor and a rotational speed were selected (rotor: cp-40; rotational speed: 25 rpm). The rotor was connected to a connecting nut, with the position adjusted, and samples were added. The Run button was pushed to start the measurement of the viscosity of the samples. According to the above experiment, the viscosity of preparation A was 5.2 centipoises, and the viscosity of preparation B was 6.0 centipoises. Surprisingly, the viscosity of preparation A was significantly lower than that of preparation B, indicating that the combination of arginine and sorbitol is more beneficial to reduce the viscosity of a high-concentration antibody preparation.

**Example 4.** Change in Protein Purity of the Liquid Preparation Comprising Anti-PCSK9 Antibody In order to inspect the stability of preparation A and preparation B, an accelerated stability test was performed. The product of preparation A and preparation B aliquoted in the plugged and capped vials were examined for accelerated stability at 40 °C ± 2 °C/60% RH ± 5% RH for 2 months. Meanwhile, the change in protein purity was assayed by SEC-HPLC and non-reduced CE-SDS as follows.

### SEC-HPLC:

The sample was diluted to a concentration of 2 mg/mL; a hydrophilic silica gel size exclusion chromatography column TSKG 3000 SWxl was used; the amount of injected protein was 100 µg; the mobile phases were 20 mmol/L Na₂HPO₄·12 H₂O, 150 mmol/L NaCl, and 200 mmol/L arginine; the pH was 6.8; the flow velocity was 0.5 mL/min; the detection wavelength was 280 nm; the column temperature was 25 °C; and the area normalization method was used for calculation.

### CE-SDS:

About 100 µg of sample was taken and added with a sample buffer (pH 7.0) to adjust the total volume to 95 µL. The reduced sample was added with 5 µL of β-mercaptoethanol, and the non-reduced sample was added with 5 µL of 250 mmol/L NEM. After mixing, the mixture was heated at 70 °C for 10 min and detected.

By SEC-HPLC, based on the percentage decrease of an SEC main peak, the changes in protein purity were assayed to be a decrease by 2.6% after 1 month and a decrease by 3.7% after 2 months for preparation A; and a decrease by 4.1% after 1 month and a decrease by 7.0% after 2 months for preparation B. Based on the percentage decrease of non-reduced CE-SDS, the changes in protein purity were assayed to be a decrease by 0.4% after 2 weeks and a decrease by 2.0% after 1 month for preparation A; and a decrease by 2.5% after 2 weeks and a decrease by 7.3% after 1 month for preparation B. It can be seen that the purity change rate of preparation A is significantly slower than that of preparation B. Relative to preparation B, preparation A has higher stability.

### Example 5. Assay of Charge Heterogeneity of the Liquid Preparation Comprising Anti-PCSK9 Antibody

At different time points, preparation A and preparation B which had undergone the accelerated stability test in Example 4 were sampled for charge heterogeneity assay. Beckman uncoated capillaries and a Beckman PA800 plus capillary electrophoresis apparatus were adopted; the sampling voltage was 0.5 psi; the sampling time was 10.0 s; the separation voltage was 30 kV; and the analysis time was 30 min. The area normalization method was used to calculate the peak area of acidic components, basic components, and the main peak as a percentage of the total peak area. The CZE main peak of preparation A dropped by 0% after 2 weeks and by 2.9% after 1 month. The CZE main peak of preparation B dropped by 2.0% after 2 weeks and by 7.6% after 1 month.

It can be seen that the charge heterogeneity change rate of preparation A is significantly slower than that of preparation B, thus the combination of arginine and sorbitol contributes to less charge heterogeneity change of the preparation.

### Example 6. Preparation of Placebo (Control Drug)

Placebo without antibody was prepared according to formula C with water as a solvent.

### Formula C

| | |
|---|---|
| Histidine | 1.5 g/L |
| Arginine | 90 mmol/L |
| Sorbitol | 3% (w/v) |
| Polysorbate 80 | 0.3 g/L |
| pH | 5.5 |

### Example 7. Pharmacokinetics Blood Sample Detection and Analysis Method of ADI-10087

(1) ADI-10087 total PK method: ADI-10087 total PK was determined by quantitative electrochemiluminescence detection of total ADI-10087 in human serum. A neutralizing antibody of ADI-10087 was coated on an MSD plate, and then a serum sample containing ADI-10087 neutralized after acidification was added, and then detection was performed by using a ruthenium-labeled polyclonal antibody of anti-ADI-10087. The ruthenium-labeled polyclonal antibody of anti-ADI-10087 formed an immune complex with the drug and neutralizing antibody of ADI-10087 coated on the plate. After appropriately incubated, the plate was washed, added with Read Buffer, and placed in an MSD plate reader; electrocatalytic chemiluminescence reaction was performed to generate luminescent signals. The intensity of the chemiluminescence signals was proportional to the content of ADI-10087 in the sample.
(2) ADI-10087 free PK method: based on the ELISA principle, the neutralizing antibody of ADI-10087 was coated and combined on a microplate, then a serum sample was added; the ADI-10087 in the serum sample could specifically bind to the coated antigen, then the neutralizing antibody of biotin-labeled ADI-10087 was added for capturing, and finally SA-HRP was added for detection. A corresponding TMB substrate of HRP enzyme was then added, the color intensity of the TMB substrate was proportional to the content of free ADI-10087, and the concentration of the monoclonal antibody of ADI-10087 in clinical serum could be detected by OD values at 450 nm or 620 nm using a microplate reader.

### Example 8. Detection Method for ADI-10087 PCSK-9

Based on the ELISA principle, ADI-10087 was coated and combined on a microplate, then a serum sample was added; PCSK-9 in the serum sample could specifically bind to the coated ADI-10087, then goat anti-human PCSK-9 polyclonal antibody was added, and finally the goat IgG HRP-conjugate antibody was added for detection. A corresponding TMB substrate of HRP enzyme was then added, the color intensity of the TMB substrate was proportional to the content of PCSK-9, and the concentration of PCSK-9 in clinical serum could be detected by OD values at 450 nm or 620 nm using a microplate reader.

### Example 9. Detection Method for Anti-drug Antibody of ADI-10087

The anti-drug antibody (ADA) of ADI-10087 in human serum was detected by semi-quantitative electrochemiluminescence. After the human serum sample was subjected to acidolysis, free ADA was captured by ADI-10087 coated on a microplate, acidolysis was performed again, the eluted ADA was mixed with biotin-labeled and ruthenium-labeled ADI-10087 for incubation to form an immune complex, and the immune complex was combined with the MSD plate coated with streptavidin. After appropriately incubated, the plate was washed, added with Read Buffer, and placed in an MSD plate reader; electrocatalytic chemiluminescence reaction was performed to generate luminescent signals. The intensity of the chemiluminescence signals was positively correlated with the content of ADA in the sample.

### Example 10. Detection Method for Neutralizing Antibody of ADI-10087

The samples determined to be positive for ADA were determined to be the neutralizing antibody (NAb) of ADI-10087 by electrochemiluminescence. After the human serum sample was subjected to acidolysis, free NAb was captured by ADI-10087 coated on a microplate, acidolysis was performed again, the eluted NAb and ruthenium-labeled ADI-10087 were mixed and incubated to form an immune complex, and then the immune complex was added into an MSD plate pre-coated with biotin-labeled PCSK-9 streptavidin for incubation. The free ruthenium-labeled drug in the analyte bound to the pre-coated biotin-labeled PCSK-9 on the plate. After appropriately incubated, the plate was washed, added with Read Buffer, and placed in an MSD plate reader; electrocatalytic chemiluminescence reaction was performed to generate luminescent signals. The intensity of the chemiluminescence signals was negatively correlated with the content of NAb in the sample.

### Example 11. Evaluation of Safety and Tolerance of Chinese Healthy Adult Subjects Receiving Single Administration of ADI-10087

### Design of Test:

A total of 58 subjects will be divided into 8 cohorts with dose escalation (25 mg SC, 75 mg SC, 75 mg IV, 150 mg SC, 300 mg SC, 450 mg SC, 450 mg IV, and 600 mg SC). The 25 mg SC cohort comprises 2 subjects who will receive a single administration of ADI-10087, and these 2 subjects, after 14 days of safety assessment, will enter clinical trials in subsequent dose cohorts. In subsequent 75 mg SC to 600 mg SC cohorts, each cohort comprises 8 subjects, wherein the first 2 subjects of the 75 mg SC group will be subjected to an open-label trial of receiving ADI-10087 75 mg SC, the last 6 subjects will receive randomly ADI-10087 (N = 4) or placebo (N = 2) in a ratio of 2:1; the other 6 cohorts of subjects will receive randomly a single administration of ADI-10087 (N = 6) or placebo (N = 2) in a ratio of 3:1.

2 subjects in the 25 mg SC cohort may enroll in the 75 mg SC cohort after completing 14 days of safety assessment after administration. All subjects in the 75 mg SC cohort may enroll in the 150 mg SC cohort after completing 14 days of safety assessment after administration. The 150 mg SC cohort may enroll the 75 mg IV cohort after enrollment. All subjects in the 150 mg SC cohort may enroll in the 300 mg SC cohort after completing 14 days of safety assessment. All subjects in the 300 mg SC cohort may enroll in the 450 mg SC cohort after completing 14 days of safety assessment after administration. All subjects in the 450 mg SC cohort may enroll in the 600 mg SC cohort after completing 14 days of safety assessment after administration. The 600 mg SC cohort may enroll the 450 mg IV cohort after enrollment.

Each subject will be followed up to day 85 (12 weeks) to evaluate safety, tolerance, PK/PD and immunogenicity of the drug.

### Demographic Characteristics of Subjects

In the safety analysis set (SS), of the 58 subjects, 44 (75.9%) subjects are males; which have an average age of 30.1 ± 5.74 years with a minimum age of 19 years and a maximum age of 44 years, an average body weight of 67.20 ± 9.419 kg with a minimum body weight of 50.9 kg and a maximum body weight of 92.4 kg, an average height of 169.12 ± 7.468 cm with the lowest height of 151.0 cm and the highest height of 186.0 cm, and an average BMI of 23.44 ± 2.309 kg/m² with a minimum BMI of 19.3 kg/m² and a maximum BMI of 27.4 kg/m². 57 subjects are Han nationality (98.3%). Of the 44 subjects in the ADI-10087 group, 43 (97.7%) subjects are Han nationality, which have an average age of 30.3 ± 5.71 years, an average height of 168.36 ± 6.992 cm, an average weight of 66.17 ± 8.289 kg, and an average BMI of 23.31 ± 2.175 kg/m². Of the 14 subjects in the placebo group, 14 (100%) subjects are Han nationality, which have an average age of 29.8 ± 6.03 years, an average height of 171.50 ± 8.716cm, an average weight of 70.43 ± 12.122 kg, and an average BMI of 23.82 ± 2.741 kg/m². The demographic characteristics of the subjects are in accordance with inclusion criteria of the study, and the demographic characteristics of the two groups of subjects are similar.

### Drug Used in the Study

The test drug used in the study was the preparation prepared as described in Example 1; the control drug used in the study was the placebo prepared as described in Example 6.

### Administration Regimens

Subjects from cohorts 1-8 were each administered with ADI-10087 or placebo at a single dose of 25 mg SC, 75 mg SC, 75 mg IV, 150 mg SC, 300 mg SC, 450 mg SC, 450 mg IV, or 600 mg SC.

***Subcutaneous (SC) Administration Regimens:*** the subjects received abdominal subcutaneous injection of the test drug or placebo. The investigators performed abdominal injections at a single administration with a subcutaneous dose of 1 mL per needle according to the actual injection volume of the test drug. The 25 mg group and the 75 mg group were injected by sucking the liquid drug with a 1 mL syringe (2 mL needle) and then injected with 1 mL needle after evacuating air. The 150 mg group and the groups with a dose greater than 150 mg were injected by sucking the liquid drug with 2 mL syringe (2 mL needle) and then injected with 1 mL needle after exhausting air.

### Intravenous (IV) Administration Regimens:

(1) 75 mg IV group: 150 mg of study drug injection (1 mL) was sucked out and added into an intravenous infusion bag of the calibrated 100 mL sterile 0.9% sodium chloride saline. The volume of intravenous administration was 50 mL and the drip time was 30 minutes.
(2) 450 mg IV group: 900 mg of study drug injection (1 mL) was sucked out and added into an intravenous infusion bag of the calibrated 200 mL sterile 0.9% sodium chloride saline. The volume of intravenous drip was 100 mL, and the drip time was 1 hour.

### Adverse Events

Adverse events (AEs) occurring during the above study were recorded, and adverse events were coded using MedDRA (version 21.0) and reported using system organ classification (SOC) and preferred term (PT). Drug-related adverse events include three categories "definitely related", "possibly related", and "unassessable". If the related-data is missing, it will be defined as a drug-related adverse event.

AEs were summarized and classified by treatment group according to SOC and PT in SOC. The results will be shown in a descending order of incidence between SOCs and within SOC. In the summary of AEs, the incidence of AEs refers to the number of subjects reporting AEs, not the number of AEs reported. In calculating the number and percentage of subjects, the AE is calculated only once if the same AE occurred multiple times in the same subject.

The AE during the screening period is the AE occurring before the first study drug administration. Treatment emergent adverse events (TEAEs) are defined as AEs that exacerbate or occur within 12 weeks (including day 85) from the first study drug administration to the last study drug administration. The generation stages of AEs which cannot be determined due to the lack of time variable are defined as TEAEs.

SOC and PT classification and summary on drug-related treatment emergent adverse events are shown in Table 1.

**Table 1: Summary on drug-related TEAE-safety analysis set by SOC and PT**

| **Systematic Organ Classification (SOC) Preferred Term (PT)** | | **25 mg SC (N=2)** | **75 mg SC (N=6)** | **75 mg IV (N=6)** | **150 mg SC (N=6)** | **300 mg SC (N=6)** | **450 mg SC (N=6)** | **450 mg IV (N=6)** | **600 mg SC (N=6)** | **Total number of subjects receiving test drug (N = 44)** | **Total number of subjects receiving Placebo (N = 14)** | **Total number of subjects (N = 58)** |
|---|---|---|---|---|---|---|---|---|---|---|---|---|
| **At least one drug-related treatment emergent adverse events** | | 1 (50.0%) | 0 | 4 (66.7%) | 3 (50.0%) | 2 (33.3%) | 0 | 3 (50.0% ) | 4 (66.7%) | 17 (38.6%) | 7 (50.0%) | 24 (41.4%) |
| **Infection and infective diseases** | | 0 | 0 | 2 (33.3%) | 2 (33.3%) | 0 | 0 | 2 (33.3%) | 3 (50.0%) | 9 (20.5%) | 2 (14.3%) | 11 (19.0%) |
| | Upper respiratory tract infection | 0 | 0 | 2 (33.3%) | 2 (33.3%) | 0 | 0 | 2 (33.3%) | 3 (50.0%) | 9 (20.5%) | 2 (14.3%) | 11 (19.0%) |
| **Investigations** | | 1 (50.0%) | 0 | 2 (33.3%) | 1 (16.7%) | 1 (16.7%) | 0 | 0 | 1 (16.7%) | 6 (13.6%) | 4 (28.6%) | 10 (17.2%) |
| | Blood creatine phosphokinase increased | 0 | 0 | 1 (16.7%) | 0 | 1 (16.7%) | 0 | 0 | 1 (16.7%) | 3 (6.8%) | 0 | 3 (5.2%) |
| | Alanine aminotransferase increased | 1 (50.0%) | 0 | 0 | 0 | 0 | 0 | 0 | 0 | 1 (2.3%) | 1 (7.1%) | 2 (3.4%) |
| | Gammaglutamyltransferase increased | 0 | 0 | 1 (16.7%) | 0 | 0 | 0 | 0 | 0 | 1 (2.3%) | 1 (7.1%) | 2 (3.4%) |
| | Aspartate aminotransferase increased | 1 (50.0%) | 0 | 0 | 0 | 0 | 0 | 0 | 0 | 1 (2.3%) | 0 | 1 (1.7%) |
| | Blood glucose increased | 0 | 0 | 0 | 0 | 0 | 0 | 0 | 0 | 0 | 1 (7.1%) | 1 (1.7%) |
| | White blood cells urine positive | 0 | 0 | 0 | 0 | 0 | 0 | 0 | 0 | 0 | 1 (7.1%) | 1 (1.7%) |
| | Protein urine present | 0 | 0 | 0 | 1 (16.7%) | 0 | 0 | 0 | 0 | 1 (2.3%) | 0 | 1 (1.7%) |
| **Blood and lymphatic system disorders** | | 0 | 0 | 0 | 0 | 0 | 0 | 1 (16.7%) | 0 | 1 (2.3%) | 1 (7.1%) | 2 (3.4%) |
| | Anemia | 0 | 0 | 0 | 0 | 0 | 0 | 1 (16.7%) | 0 | 1 (2.3%) | 1 (7.1%) | 2 (3.4%) |
| **Musculoskeletal and connective tissue disorders** | | 0 | 0 | 1 (16.7%) | 0 | 0 | 0 | 0 | 0 | 1 (2.3%) | 1 (7.1%) | 2 (3.4%) |
| | Bone pain | 0 | 0 | 1 (16.7%) | 0 | 0 | 0 | 0 | 0 | 1 (2.3%) | 0 | 1 (1.7%) |
| | Musculoskeletal pain | 0 | 0 | 0 | 0 | 0 | 0 | 0 | 0 | 0 | 1 (7.1%) | 1 (1.7%) |
| **Skin and subcutaneous tissue disorders** | | 0 | 0 | 0 | 0 | 0 | 0 | 0 | 1 (16.7%) | 1 (2.3%) | 1 (7.1%) | 2 (3.4%) |
| | Allergic dermatitis | 0 | 0 | 0 | 0 | 0 | 0 | 0 | 1 (16.7%) | 1 (2.3%) | 0 | 1 (1.7%) |
| | Papule | 0 | 0 | 0 | 0 | 0 | 0 | 0 | 0 | 0 | 1 (7.1%) | 1 (1.7%) |
| **Gastrointestinal disorders** | | 0 | 0 | 0 | 0 | 0 | 0 | 1 (16.7%) | 0 | 1 (2.3%) | 0 | 1 (1.7%) |
| | Gingival disease | 0 | 0 | 0 | 0 | 0 | 0 | 1 (16.7%) | 0 | 1 (2.3%) | 0 | 1 (1.7%) |
| | Mouth ulcer | 0 | 0 | 0 | 0 | 0 | 0 | 1 (16.7%) | 0 | 1 (2.3%) | 0 | 1 (1.7%) |
| **General disorders and administration site conditions** | | 0 | 0 | 0 | 0 | 1 (16.7%) | 0 | 0 | 0 | 1 (2.3%) | 0 | 1 (1.7%) |
| | Injection site erythema | 0 | 0 | 0 | 0 | 1 (16.7%) | 0 | 0 | 0 | 1 (2.3%) | 0 | 1 (1.7%) |
| **Respiratory, thoracic and mediastinal disorders** | | 1 (50.0%) | 0 | 0 | 0 | 0 | 0 | 0 | 0 | 1 (2.3%) | 0 | 1 (1.7%) |
| | Cough | 1 (50.0%) | 0 | 0 | 0 | 0 | 0 | 0 | 0 | 1 (2.3%) | 0 | 1 (1.7%) |

The incidence rate of treatment emergent adverse events is 52.3% and the incidence rate of drug-related treatment emergent adverse events is 38.6% in the ADI-10087 group; the incidence rate of treatment emergent adverse events is 57.1% and the incidence rate of drug-related treatment emergent adverse events is 50.0% in the placebo group. The incidence rate of at least one adverse event of specific interest (liver event, muscle event, allergic reaction, and injection site reaction) in the ADI-10087 and placebo groups were 13.6% and 28.6%, respectively, without severe adverse events, drug-related severe adverse events, adverse events leading to withdrawal from study and adverse events leading to death occurring. The most common drug-related treatment emergent adverse event in the ADI-10087 group was upper respiratory tract infection (20.5%) and blood creatine phosphokinase increased (6.8%); the most common drug-related treatment emergent adverse event in the placebo group was upper respiratory tract infection (14.3%).

The drug-related adverse events in the ADI-10087 group were mild to moderate without special treatment and improved after corrective treatment. In particular, no adverse events occurred in the SC group at a dose of up to 450 mg.

### Immunogenicity Assessment

Immunogenicity assessment was based on immunogenicity analysis set (IS) with evaluation indices of anti-ADI-10087 antibody (ADA) and neutralizing antibody (NAb). The proportion of subjects who produced anti-ADI-10087 antibody and neutralizing antibody (NAb) was summarized for each dose group, and subjects who were positive for ADI-10087 antibody (ADA)/neutralizing antibody (NAb) after drug administration were listed.

The production of anti-drug antibody (ADA) and neutralizing antibody (NAb) in the serum was determined by immunogenicity assay. (1) Sampling time points for anti-drug antibody and neutralizing antibody detection: 1 hour before administration; 336 hours (day 15), 672 hours (day 29), 1344 hours (day 57), 2016 hours (day 85) after administration

Immunogenicity blood sampling point: within 1 hour before administration; 336 hours (± 12 h) (day 15), 672 hours (± 24 h) (day 29), 1344 hours (± 48 h) (day 57), 2016 hours (± 48 h) (day 85) after administration.

No subject was detected positive for anti-ADI-10087 antibody (ADA) after administration, except for 1 subject (1.7%) in the placebo group was positive for ADA before administration at baseline. The subject was detected negative for neutralizing antibody (NAb). Therefore, no ADA and NAb of subjects in the ADI-10087 group and the placebo group was positive after administration, which has good safety.

### Example 12. Evaluation of Pharmacokinetics of Chinese Healthy Adult Subjects Receiving Single Administration of ADI-10087

In the study described in Example 11 above, PK blood samples were collected at the following time points:
1 hour before administration; 4 hours after administration; 24 hours (day 2), 48 hours (day 3), 72 hours (day 4), 96 hours (day 5), 144 hours (day 7), 168 hours (day 8), 240 hours (day 11), 336 hours (day 15), 504 hours (day 22), 672 hours (day 29), 840 hours (day 36), 1008 hours (day 43), 1344 hours (day 57), 1680 hours (day 71), 2016 hours (day 85) after injection administration.

Pharmacokinetic parameters for total ADI-10087 and free ADI-10087 were calculated at actual blood collection times using non-compartmental model analysis (NCA) using WinNonlin Professional software, including: Tₘₐₓ, Cₘₐₓ, AUC, V, t_{1/2}, and CL, to fully reflect the distribution and elimination characteristics of the drug in human body, and the main parameters are shown in the following Tables 2 and 3.

**Table 2. Summary of total ADI-10087 PK parameters of healthy subjects after administration of various doses of ADI-10087 ^{a}**

| **Dose group** | **Cₘₐₓ(µg/mL)** | **Tₘₐₓ (h)** | **AUC₀₋ₗₐₛₜ (µg•day/mL)** | **AUC _{%extrap} %** | **AUC_{0-inf} (µg•day/mL)** | **λ_{Z} (1/h)** | **CL(/F) (mL/h)** | **^{I} Vz(/F) (mL)** | **t_{1/2} (day)** | **Vₛₛ^{e} (mL)** |
|---|---|---|---|---|---|---|---|---|---|---|
| 25mg SC^{b} (n = 2) | 2.25 | 72.0 (72,0,72,0) | 25.2 | 3.99 | 26.2 | 0.006 | 40.2 | 7380.3 | 5.24 | |
| 75mg IV (n-6) | 26.7 ±3.24 12.1% | 4.00 (0.500,4.00) | 185.0±40.5 21.9% | 0.433±0.213 49.2% | 185.8±40.4 21.7% | 0.007 ±0.001 7.75% | 17.3±2.85 16.4% | 2600.8 +540.8 20.8% | 4.32 ±0.452 10.5% | 2960.3 +299.0 10.1% |
| 75mg SC (n = 6) | 7.59±1.99 26.2% | 119.9 (72.0,167.8) | 106.7±23.7 22.2% | 1.15±0.385 33.5% | 107.9±23.9 22.2% | 0.006±0.001 12.9% | 30.2±6.81 22.5% | 5298.7 ±1728.1 32.6% | 5.01 +0.608 12.2% | |
| 150mg SC (n = 6) | 17.9±3.18 17.8% | 96.0 (48.0,144.9) | 274.4±84.4 30.7% | 0.540±0.210 38.9% | 275.9 ±84.8 30.7% | 0.006±0.001 18.2% | 24.0±5.11 21.3% | 4159.2±677.5 16.3% | 5.14±0.822 16.0% | |
| 300mg SC (n = 6) | 29.1±11.6 39.7% | 120.1 (72.0,335.4) | 661.2 ±227.2 34.4% | 0.197±0.164 83.1% | 662.3 ±226.9 34.3% | 0.005 ±0.001 22.6% | 21.4±9.30 43.4% | 4034.6±1365.5 33.8% | 5.64 ±1.06 18.8% | |
| 450mg IV (n = 6)^{d} | 123.5±15.7 12.7% | 4.00 (4.00,4.00) | 2068.3±689.8 33.4% | 0.175 +0.205 117.2% | 2073.1±696.4 33.6% | 0.005 ±0.000 9.32% | 9.81 ±2.95 30.1% | 2138.2±568.7 26.6% | 6.43±0.917 14.3% | 4306.8 ±731.1 17.0% |
| 450mg SC (n = 6) | 48.6±14.0 28.8% | 143.6 (72.0,168.1) | 1147.8±136.6 11.9% | 0.126±0.041 32.7% | 1149.3±136.8 11.9% | 0.005±0.001 15.6% | 16.5±2.19 13.3% | 3499.2±886.8 25.3% | 6.06+0.942 15.6% | |
| 600mg SC (n = 6) | 64.9±15.9 24.4% | 96.0 (96.0,337.9) | 2068.8±561.9 27.2% | 0.468 ±0.449 95.9% | 2079.8±569.9 27.4% | 0.004±0.001 18.1% | 13.0±4.57 35.0% | 3223.9 ±1094.4 33.9% | 7.22 +1.01 14.0% | |

| | | | | | | | | | | |
|---|---|---|---|---|---|---|---|---|---|---|
| Cₘₐₓ: peak concentration; Tₘₐₓ: peak time; AUC₀₋ₗₐₛₜ: area under drug-time curve from time 0 to the last measurable concentration time point; AUC__{%extrap}: percentage of the extrapolated area under drug-time curve; AUC_{0-inf}: area under drug-time curve from time 0 to infinity; λ_{z}: elimination rate constant; CL(/F): clearance (apparent clearance); V_{z}(/F): distribution volume (apparent distribution volume); t_{1/2}: half-life; Vₛₛ: homeostatic distribution volume; SC: subcutaneous administration; IV: intravenous drip. ^{a}: PK parameters were expressed as arithmetic mean + standard deviation (coefficient of variation %) besides that Tₘₐₓ was expressed as median (minimum, maximum). ^{b}: only the arithmetic mean was reported due to a small sample size. ^{c}: Vₛₛ was reported only on intravenous drip administration. ^{d}: all parameters of subject 01301 in the 450 mg IV group were missing 504 h after administration, failing to calculate AUC₀-_{LAST} and the parameters related to λ_{z} (AUC_{0-inf}, AUC _{-extrap%}, λ_{z}, CL, Vz, Vₛₛ, and t_{1/2}). For these parameters, n = 5. | | | | | | | | | | |

**Table 3. Summary of free ADI-10087 PK parameters of healthy subjects after administration of various doses of ADI-10087 ^{a}**

| **Dose group** | **Cₘₐₓ (µg/mL)** | **Tₘₐₓ (h)** | **AUC₀₋ₗₐₛₜ (µg•day/mL)** | **AUC _{%extrap} (%)** | **AUS_{0-inf} (µg•day/mL)** | **λz (1/h)** | ^{I} **CL(/F) (mL/h)** | **Vz(/F) (mL)** | **t_{1/2} (day)** | **Vₛₛ^{c}** ' **(mL)** | R **Cₘₐₓ** | ' **R_{AUC0-last}** | ^{I} **R_{AUC0-inf}** |
|---|---|---|---|---|---|---|---|---|---|---|---|---|---|
| 25mg (n = 2) | - | - | - | - | - | - | - | - | - | | - | - | - |
| 75mg IV (n = 6) | 30.3±3.98 13.1% | 0.500 (0.500,4.00) | 127.5±25.0 19.6% | 4.57±2.27 49.6% | 133.8±27.4 20.4% | 0.011±0.003 31.5% | 24.1±4.58 19.0% | 2274.4±528.8 23.2% | 2.82±0.860 30.5% | 2459.1±397.3 16.2% | 1.14±0.088 4.93% | 0.695 ±0.083 11.9% | 0.725⁺0.088 12.1% |
| 75mg SC (n = 6)^{d} | 5.87±2.45 41.7% | 60.0 (47.9,96.0) | 45.4±13.2 29.1% | 7.68±2.67 34.7% | 46.6±14.2 30.4% | 0.009±0.001 13.6% | 74.6±31.4 42.1% | 8583.7±4589.8 53.5% | 3.26+0.420 12.9% | | 0.744±0.096 19.5% | 0.422+0.083 19.8% | 0.470±0.096 20.4% |
| 150mg SC (n = 6)^{e} | 17.3±6.20 35.9% | 72.0 (48.0,144.8) | 178.1±83.4 46.8% | 3.04±1.45 47.7% | 219.6±105.7 48.1% | 0.008±0.002 25.0% | 32.5±12.8 39.2% | 4039.9±1511.7 37.4% | 3.72±0.836 22.5% | | 0.986±0.168 36.9% | 0.641±0.177 27.6% | 0.706±0.168 23.8% |
| 300mg SC (n = 6) | 31.8±14.3 45.1% | 119.9 (96.0,239.6) | 553.9±237.0 42.8% | 3.68±4.23 114.9% | 569.3±233.8 41.1% | 0.006±0.001 20.0% | 26.8±14.6 54.6% | 4946.3±3234.1 65.4% | 5.22±1.24 23.7% | | 1.07±0.119 9.19% | 0.812±0.133 16.4% | 0.840±0.119 14.2% |
| 450mg IV (n = 6)^{f} | 185.8±30.8 16.6% | 4.00 (4.00,4.00) | 2173.8±764.8 35.2% | 1.06±1.15 108.4% | 2193.7±759.8 34.6% | 0.005+0.002 34.4% | 9.41±3.28 34.9% | 2146.0±1178.4 54.9% | 6.67±2.89 43.3% | 3034,4+704.0 23.2% | 1.50±0.079 11.0% | 1.04+0.079 7.54% | 1.05±0.079 7.48% |
| 450mg SC (n = 6)^{g} | 58.1±21.3 36.6% | 144.4 (95.8,239.8) | 973.9±145.5 14.9% | 2.96±1.72 57.9% | 1034.4±126.7 12.3% | 0.004±0.001 19.9% | 18.4±2.43 13.2% | 4666.3±1706.4 36.6% | 7.21±1,68 23.4% | | 1.19±0.049 19.6% | 0.847±0.057 6.72% | 0.879±0.049 5.60% |
| 600mg SC (n = 6) | 70.2±30.9 44.1% | 239.5 (143.6,503.8) | 1542.2±486.9 31.6% | 2.03±2.18 107.4% | 1568.0±478.8 30.5% | 0.004±0.001 15.8% | 17.4±6.04 34.7% | 4673.5±2817.9 60.3% | 7.36±1.57 21.3% | | 1.06±0.116 26.5% | 0.746±0.114 15.3% | 0.758±0.116 15.3% |

| | | | | | | | | | | | | | |
|---|---|---|---|---|---|---|---|---|---|---|---|---|---|
| Cₘₐₓ: peak concentration; Tₘₐₓ: peak time; AUC₀₋ₗₐₛₜ: area under drug-time curve from time 0 to the last measurable concentration time point; AUC __{%extrap}: percentage of the extrapolated area under drug-time curve; AUC_{0-inf}: area under drug-time curve from time 0 to infinity; λ_{z}: elimination rate constant; CL(/F): clearance (apparent clearance); V_{z}(/F): distribution volume (apparent distribution volume); t_{1/2}: half-life; Vₛₛ: homeostatic distribution volume; R_{Cmax,} R_{AUC0-last}ANDR_{AUC0-inf} are ratio of Cₘₐₓ, AUC₀-ₗₐₛₜ and AUC_{0-inf}of free ADI-10087 to total ADI-10087, respectively; SC: subcutaneous administration; IV: intravenous drip. ^{a}: PK parameters were expressed as arithmetic mean + standard deviation (coefficient of variation %) besides that Tₘₐₓ was expressed as median (minimum, maximum). ^{b}: ADI-10087 free concentration was lower in the 25 mg SC dose group and the plasma concentration data obtained were less (no more than 3 plasma concentrations per subject) and insufficient for assessment of PK parameters. ^{c}: Vₛₛ was reported only on intravenous drip administration. ^{d}: λ_{z} of subject 01047 in the 75 mg SC group cannot be assessed accurately, failing to calculate AUC₀-ₗₐₛₜ and the parameters related to λ_{z} (AUC_{0-inf}, AUC __{%extrap}, λ_{z}, CL/F, V_{z}/F, and t_{1/2}). For these parameters, n = 5. ^{e}: λ_{z} of subjects 01096, 01101 and 01122 in the 150 mg SC group cannot be assessed accurately, failing to calculate the parameters related to λ_{z} (AUC_{0-inf}, AUC _{%extrap}, λ_{z}, CL/F, V_{z}/F, and t_{1/2}). For these parameters, n = 3. ^{f}: all parameters of subject 01301 in the 450 mg IV group were missing 504 h after administration, failing to calculate AUC₀-ₗₐₛₜ and the parameters related to λ_{z} (AUC_{0-inf}, AUC _{%extrap}, λ_{z}, CL, V_{z}, Vₛₛ, and t_{1/2}). For these parameters, n = 5. ^{g}: λ_{z} of subject 01192 in the 450 mg SC group cannot be assessed accurately, failing to calculate the parameters related to λ_{z} (AUC_{0-inf}, AUC __{%extrap}, λ_{z}, CL/F, V_{z}/F, and t_{1/2}). For these parameters, n = 5. | | | | | | | | | | | | | |

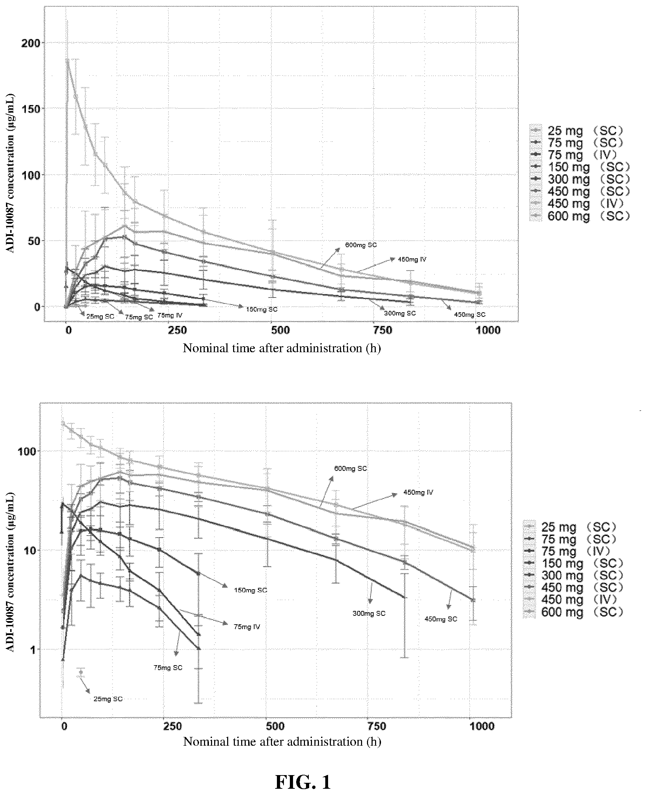

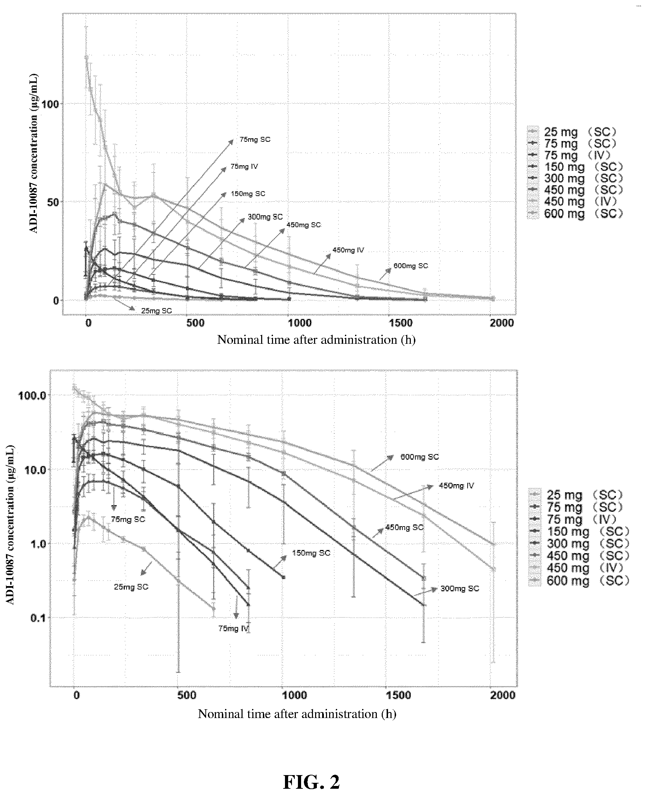

The average total concentration-time curve of ADI-10087 after administration of ADI-10087 to subjects in each dose group is shown in FIG. 2, and the average free concentration-time curve of ADI-10087 in each dose group is shown in FIG. 1.

Pharmacokinetic assessments were based on pharmacokinetic concentration set (PKCS) which was analyzed with 44 subjects' data. After a single abdominal subcutaneous injection of 25-600 mg of ADI-10087, the average apparent distribution volume (V_{z/F}) of total ADI-10087 was about 3.2-7.4 L, the average apparent clearance (CL/F) was about 13.0-40.2 mL/h, and the average t_{1/2} was about 5-7 days; the average V _{z/F} of free ADI-10087 was about 4.0-8.6 L, the average CL/F was 17.4-74.6 mL/h, and the average t_{1/2} was about 3-7 days. As the dose increased, Vz/F and CL/F continuously decreased and t_{1/2} gradually prolonged.

After a single abdominal subcutaneous injection of 25-600 mg of ADI-10087, Tₘₐₓ of total ADI-10087 in serum was about 3-6 days, and Tₘₐₓ of free ADI-10087 was about 2.5-10 days. It is indicated that ADI-10087 in the abdomen takes time to absorb, and as the dose increases, it takes longer to reach absorption peak.

Within a dose range of a single administration of 25-600 mg of ADI-10087, the increase proportions of Cmax, AUC₀₋ₗₐₛₜ and AUC_{0-∞of} total ADI-10087 and free ADI-10087 were larger than the increase proportion of the dose, and the non-linear kinetic characteristic was presented.

After a single intravenous drip of 75 mg and 450 mg, the average V_{z} of total ADI-10087 was about 2.6 L and 2.1 L, respectively, the average Vₛₛ was about 3.0 L and 4.3 L, respectively, the average CL was 17.3 mL/h and 9.81 mL/h, respectively, and the average t_{1/2} was about 4 days and 6 days, respectively; the average V_{z} of free ADI-10087 was about 2.3 L and 2.1 L, respectively, the average Vₛₛ was about 2.5 L and 3.0 L, respectively, the average CL was 24.1 mL/h and 9.41 mL/h, respectively, and the average t_{1/2} was about 3 and 7 days, respectively. Also, as the dose increased, CL decreased and t_{1/2} prolonged. The absolute bioavailability of ADI-10087 for abdominal subcutaneous injection was about 58% (based on total ADI-10087) at a dose range of 75-450 mg.

After abdominal subcutaneous injection or intravenous drip of 75 mg and 450 mg to healthy subjects, the corresponding AUC_{0-∞}geometric mean of total ADI-10087 and free ADI-10087 and the corresponding absolute bioavailability estimates for subcutaneous administration are shown in Table 4.

**Table 4. Absolute bioavailability for abdominal subcutaneous injection of 75 mg and 450 mg of ADI-10087**

| **Dose group** | **Analyte** | **AUC₀-_{inf} for abdominal subcutaneous injection administration Geometric mean (geometric CV%) (µg•day/mL) (N = 6)** | **AUC₀-_{inf} for intravenous drip administration Geometric mean (geometric CV%) (µg•day/mL) (N = 5)^{a}** | **Subcutaneous administration Absolute bioavailability (F)** |
|---|---|---|---|---|
| 75 mg | Total ADI-10087 | 105.7(22.7%) | 182.7(19.2%) | 57.8% |
| | Free ADI-10087 | 44.5(37.7) | 131.7(19.8%) | 33.8% |
| 450 mg | Total ADI-10087 | 1142.0( 12.6%) | 1988.4(32.6% ) | 57.5% |
| | Free ADI-10087 | 1027.9(12.8% ) | 2090.4(36.0% ) | 49.2% |

| | | | | |
|---|---|---|---|---|
| AUC_{0-inf}: area under drug-time curve from time 0 to infinity. | | | | |

It can be seen that when evaluated based on total ADI-10087, the absolute bioavailability of subcutaneous administration in healthy subjects was similar at doses of 75 mg and 450 mg, being 57.8% and 57.5%, respectively, which indicates that the absolute bioavailability of subcutaneous administration was substantially consistent in the dose range of 75-450 mg.

When evaluated based on free ADI-10087, the absolute bioavailability of subcutaneous administration in healthy subjects was 33.8% and 49.2% at doses of 75 mg and 450 mg, respectively; which is slightly lower than the evaluation result of total ADI-10087.

The graphical method was used to analyze the correlation between PK parameters (Cₘₐₓ, AUC₀₋ₗₐₛₜ and AUC_{0-∞}) and the dose in the 25-600 mg dose group with abdominal subcutaneous injection.

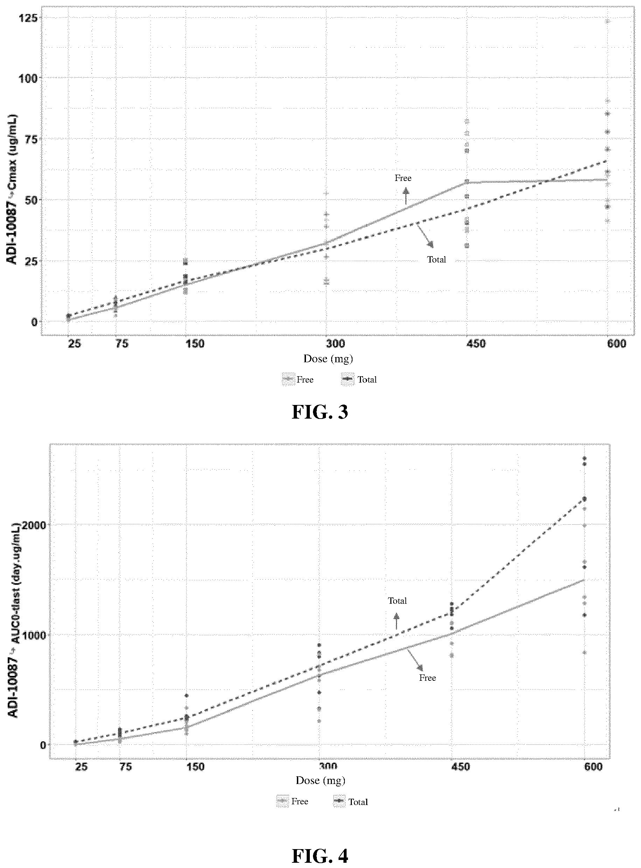

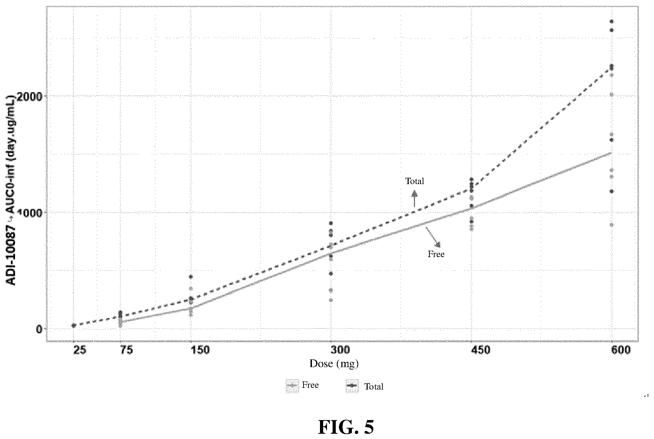

Scatter plots of Cₘₐₓ, AUC₀₋ₗₐₛₜ and AUC₀-_{inf} of total ADI-10087 and free ADI-10087 after abdominal subcutaneous injection of 25-600 mg to healthy subjects that vary with doses are shown in FIGs. 3-5, respectively. The results show that in the dose range of 25-600 mg, Cₘₐₓ and AUC of total ADI-10087 and free ADI-10087 increased along with the increase of dose, but the increase proportion was larger than the increase proportion of the dose, and the ADI-10087 presented non-linear kinetic characteristics. For example, the average Cₘₐₓ of total ADI-10087 increased by a ratio of about 1:3.4:8.0:13:22:29 and the average AUC₀₋ₗₐₛₜ increased by a ratio of about 1:4.2:11:26:46:82 relative to the fold increase in 25-600 mg dose (1:3:6:12:18:24). For free ADI-10087, the 25 mg group failed to report PK parameters due to the lower concentration; in the dose range of 75-600 mg (increasing multiple of 1:2:4:6:8), the average Cₘₐₓ of free ADI-10087 increased by about 1:2.9:5.4:10:12 and the average AUC₀₋ₗₐₛₜ increased by about 1:3.9:12:21:34.

Only two doses, 75 mg and 450 mg, were evaluated by intravenous drip. When the dose of intravenous drip increased 6-fold, the average Cₘₐₓ and AUC₀₋ₗₐₛₜ of total ADI-10087 increased 4.6-fold and 11-fold, respectively; the average Cₘₐₓ and AUC₀-ₗₐₛₜ of free ADI-10087 increased 6.1-fold and 17-fold, respectively.

### Example 13. Evaluation of Pharmacodynamics of Chinese Healthy Adult Subjects Receiving Single Administration of ADI-10087

In the study described in Example 11 above, blood samples were collected at the following time points:1 hour before administration; 4 hours after administration; 24 hours (day 2), 48 hours (day 3), 72 hours (day 4), 96 hours (day 5), 144 hours (day 7), 168 hours (day 8), 240 hours (day 11), 336 hours (day 15), 504 hours (day 22), 672 hours (day 29), 840 hours (day 36), 1008 hours (day 43), 1344 hours (day 57), 1680 hours (day 71), 2016 hours (day 85) after injection administration. The levels of PCSK-9, LDL-C, total cholesterol (TC), triglycerides (TG), high-density lipoprotein cholesterol (HDL-C), lipoprotein a (Lp(a)), very low-density lipoprotein cholesterol (VLDL-C), non-high-density lipoprotein cholesterol (non-HDL-C), apolipoprotein A1 (ApoA1), apolipoprotein B (ApoB), and the like were determined according to the methods disclosed herein or well known in the art, and the graphs were plotted.

### (1) Change of serum free PCSK-9 concentration relative to baseline

Blood samples will be collected at designated time points by the central laboratory to test serum free PCSK-9 concentrations. Pharmacodynamic evaluation will be based on pharmacodynamic analysis set (PDS), and the observed values at each time point and the change rate (%) of serum free PCSK-9 concentration relative to baseline will be summarized and listed. Baseline was defined as the observed PCSK-9 concentration value before administration. Change rate relative to baseline (%) = 100 × (observed concentration value after administration - baseline value)/baseline value. Individual serum free PCSK-9 concentration, average value of PCSK-9 concentration for each dose group, and change rate relative to baseline will be plotted for drug concentration-time curve (including a linear concentration graph and a logarithmic concentration graph) based on dose groups.

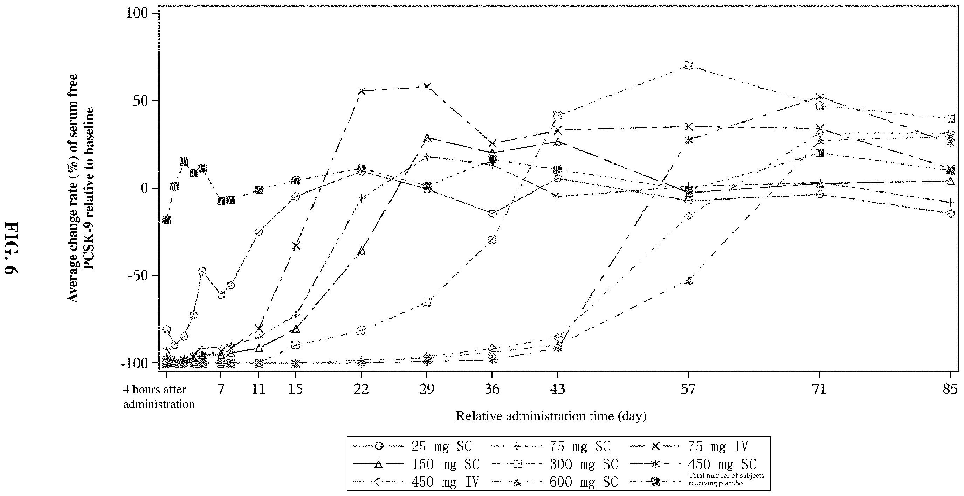

Change rate in serum free PCSK-9 concentration relative to baseline is shown in FIG. 6. The results show that: the average duration of PCSK-9 decrease prolonged with increasing dose. 24 hours after administration, the average maximum decrease of PCSK-9 in each group was 90%-100%; 2 weeks after administration, PCSK-9 decreased by > 70% on average in ADI-10087 75 mg SC group and 150 mg and above groups; 4 weeks after administration, PCSK-9 decreased by > 60% on average in ADI-10087 300 mg and above SC groups and IV group; 6 weeks after administration, PCSK-9 decreased by > 80% on average in ADI-10087 450 mg SC group, 450 mg IV group and 600 mg SC group; 8 weeks after administration, PCSK-9 decreased by > 50% on average in ADI-10087 600 mg SC group.

### (2) Least squares mean of LDL-C changes relative to baseline

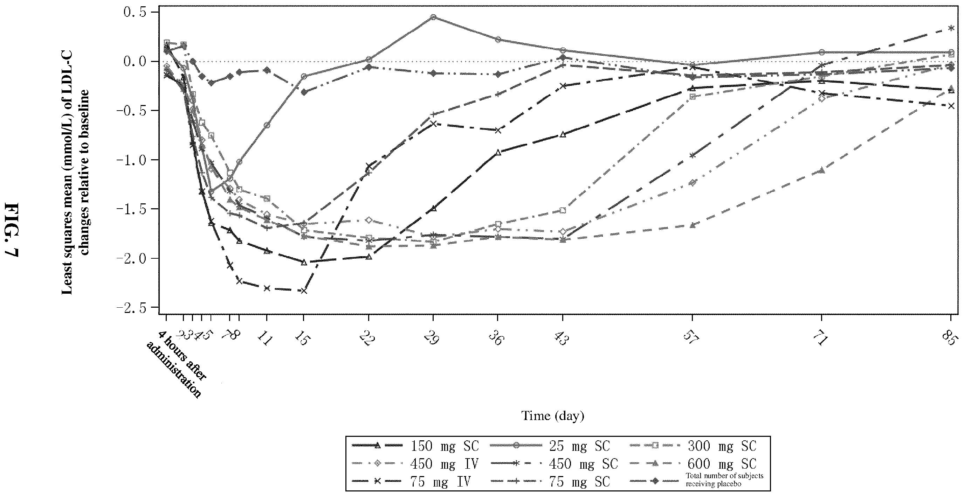

At each evaluation time point, the comparison of changes in serum LDL-C relative to baseline for each test dose group and placebo group was analyzed using a mixed effects models for repeated measures (MMRM), and the analysis results included the least squares means of the change values, the means of differences between groups, and 95% confidence interval and P value. From the results of the model analysis, the P value of most test drug dose groups compared with placebo group was < 0.05 within one month after administration. And over time, the P value of the low dose group compared with the placebo group became > 0.05. By "day 29", the P value of the 150 mg and above groups compared with placebo group was < 0.05; by "day 57", the P value of only the 450 mg and above groups compared with placebo group was < 0.05; by "day 71", the P value of only the 600 mg dose group compared with placebo group was < 0.05; by "day 85", the P values of all test drug dose groups compared with placebo groups were > 0.05. The least squares means of LDL-C changes relative to baseline at each efficacy evaluation time point for each dose group are shown in FIG. 7.

### (3) Average change rate of LDL-C concentration relative to baseline

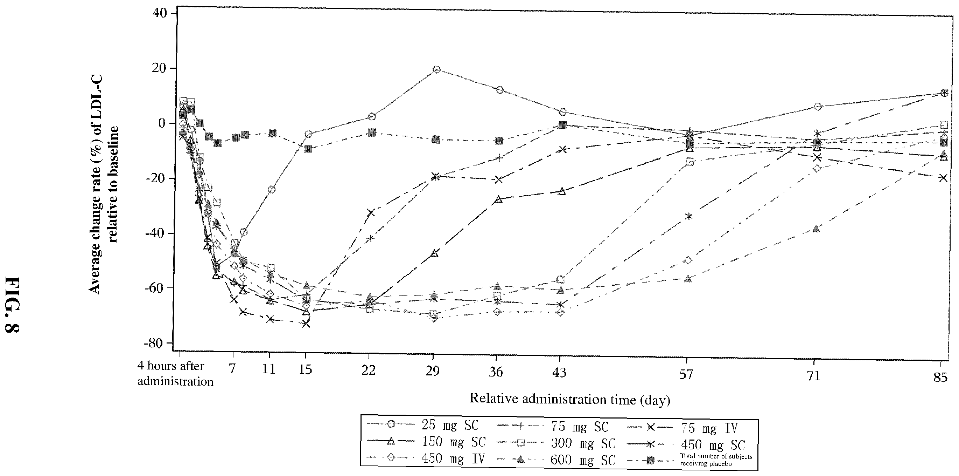

The average change rate of LDL-C concentration relative to baseline is shown in FIG. 8. The results show that: the duration of serum LDL-C decrease was dose-dependent with ADI-10087. The near-maximal LDL-C decrease first occurred on day 5. On day 15, LDL-C in each dose group was close to a maximum decrease. The average maximum decrease of LDL-C in each group of ADI-10087 subcutaneous injection was between 51.7%-72.1%. 7 days after administration, the average decrease of LDL-C in the ADI-10087 75 mg and 150 mg groups was > 50%, the average decrease of LDL-C of 75 mg group was > 50% that maintained for 15 days, and the average decrease of LDL-C of 150 mg group was maintained for 22 days. On day 11 after administration, the average decrease of LDL-C in the ADI-10087 300 mg and above groups was > 50%, which may be maintained to at least day 43 (week 6). On day 57 (8 weeks) after administration, the average decrease of LDL-C in the ADI-10087 600 mg SC group was > 50%. On day 71 (10 weeks) after administration, the average decrease of LDL-C in the ADI-10087 600 mg SC group was > 30%.

Compared with Evolocumab as control, it was found that the maximum decrease and duration of LDL-C decrease of ADI-10087was > Evolocumab at the same dose.

### (4) Area under LDL-C level curve

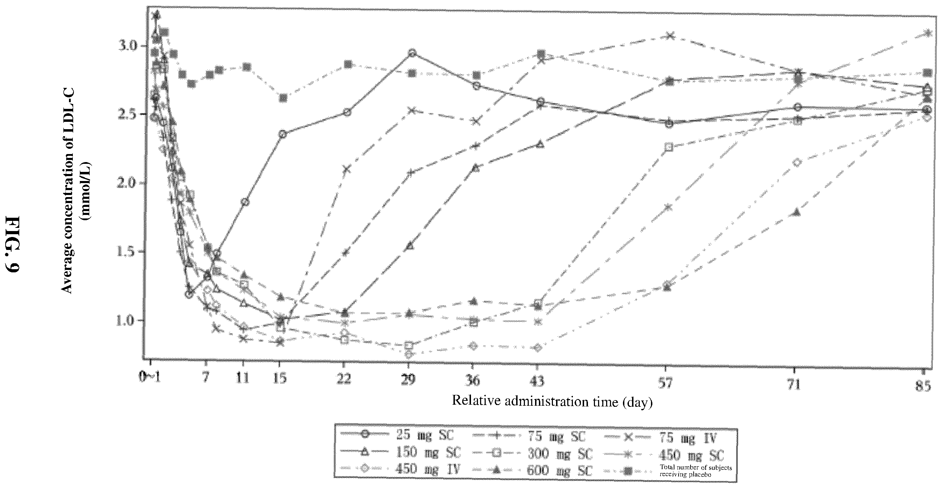

The average LDL-C concentration-time plot is shown in FIG. 9.

The area under the LDL-C level curve was calculated using a trapezoidal method. The area under the LDL-C curve for subjects who had previously withdrawn was also calculated using the trapezoidal method based on concentration data before withdraw. In area under LDL-C level curve, the geometric mean of the total test drug groups was 3522.433 and the geometric coefficient of variation was 44.3; the geometric mean of the total placebo groups was 5609.877 and the geometric coefficient of variation was 20.1. The area under the curve for the 75 mg IV group in the test drug groups was the largest, the geometric mean was 4709.056, and the geometric coefficient of variation was 18.0; the area under the curve for 450 mg IV group was the smallest, the geometric mean was 1978.572, and the geometric coefficient of variation was 99.0.

### (5) Change rate of concentration of total cholesterol (TC) relative to baseline

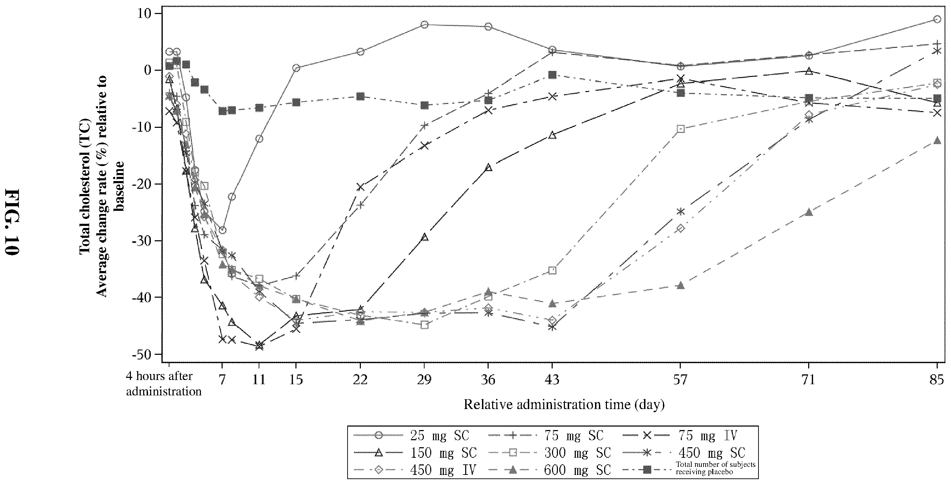

The change rate of concentration of total cholesterol (TC) relative to baseline is shown in FIG. 10. The results show that: the average decrease duration of total cholesterol prolonged with increasing dose. In the ADI-10087 75 mg and above dose groups, the average maximum decrease of total cholesterol ranged from 37.9% to 48.6%, which was close to the maximum 11 days after administration.

### (6) Change rate of concentration of apolipoprotein B (ApoB) relative to baseline

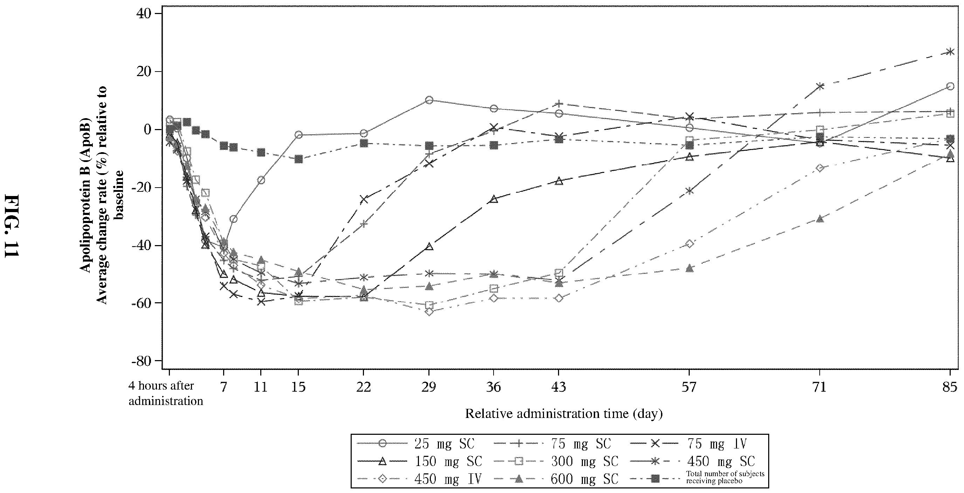

The change rate of concentration of apolipoprotein B (ApoB) relative to baseline is shown in FIG. 11. The results show that: the average duration of ApoB decrease prolonged with increasing dose. In the ADI-10087 75 mg and above dose groups, the average maximum decrease of total cholesterol ranged from 52.1% to 62.8%, which was close to the maximum 11 days after administration.

### (7) Change rate of concentration of non-high-density lipoprotein cholesterol (Non-HDL-C) relative to baseline

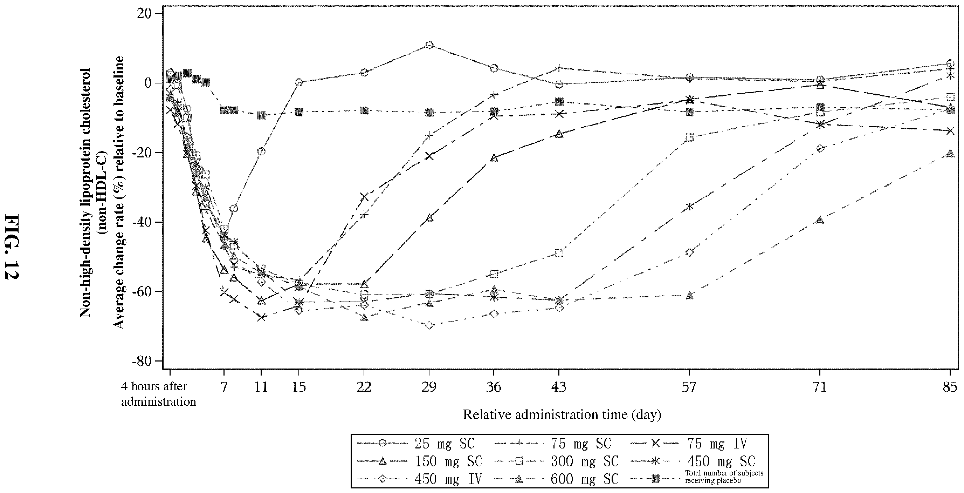

The change rate of concentration of non-high-density lipoprotein cholesterol (Non-HDL-C) relative to baseline is shown in FIG. 12. The results show that: the average duration of Non-HDL-C decrease prolonged with increasing dose. In the ADI-10087 75 mg and above dose groups, the average maximum decrease of total cholesterol ranged from 56.8% to 69.7%, which was close to the maximum 11 days after administration.

### (8) Change rate of average concentration of lipoprotein a (LP(a)) relative to baseline

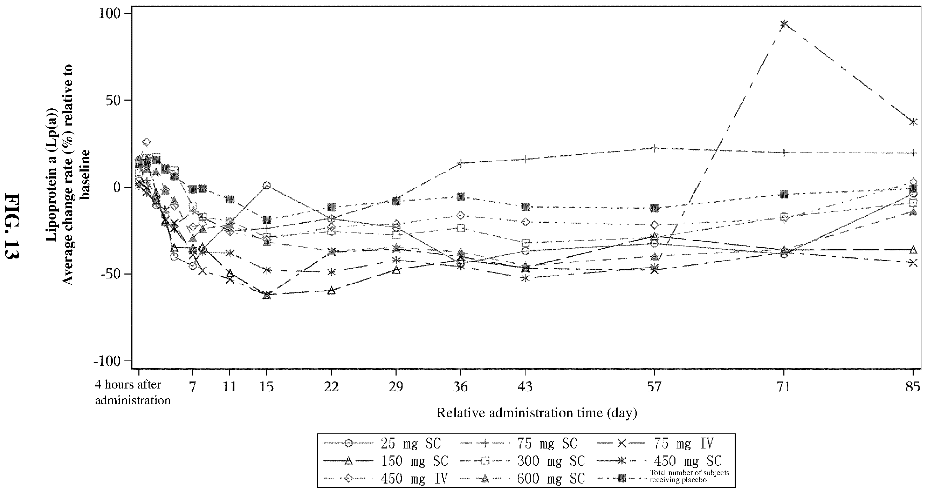

The change rate of average concentration of lipoprotein a (LP(a)) relative to baseline is shown in FIG. 13. 7 days after administration, LP(a) of each dose groups of ADI-10087 decreased, and the average maximum decrease in each group ranged from 24.9% to 62.3%.

### Example 14. Evaluation of Pharmacokinetics of Multiple Dose Repeated Administrations of ADI-10087 in Chinese Population with Hypercholesterolemia

### 1. Selection of study population

The enrolled subjects must meet all of the following criteria simultaneously:
(1) providing a signed and dated informed consent;
(2) the male or female with the age ≥ 18 and ≤ 70 years old when screening;
(3) BMI > 18 kg/m² and < 30 kg/m²;
(4) diagnosed with hyperlipidemia, and treated with moderate-intensity or more dose of statin for at least 4 weeks (specifically refer to Guidelines for the Prevention and Treatment of Dyslipidemia in Chinese Adults 2016*);*
(5) fasting LDL-C ≥ 100 mg/dL (2.6 mmol/L) and ≤ 220 mg/dL (5.7 mmol/L)when screening;
(6) fasting triglyceride ≤ 400 mg (4.5 mmol/L) when screening; and
(7) having a willingness to coordinate to complete all steps in study and study intervention cycle.

### 2. Design of test

A total of 60 patients meeting inclusion criteria were randomly evenly distributed into the following different dose groups which received separately: 1 subcutaneous injection of 75 mg or 140 mg of ADI-10087 or placebo every 2 weeks, or 1 subcutaneous injection of 300 mg or 420 mg of ADI-10087 or placebo every 4 weeks, or 1 subcutaneous injection of 450 mg or 600 mg ADI-10087 or placebo every 6 weeks. ADI-10087 and placebo were randomized according to 4:1 in each dose. Treatment continued for 12 weeks, after which the results were analyzed.

### 3. Drugs used in the study

The test drug used in the study was the preparation prepared as described in Example 1; the control drug used in the study was the placebo prepared as described in Example 6.

### 4. Administration regimens

Abdominal subcutaneous injection of test drug or placebo

### 5. Sample collection and processing

PK/PD (PCSK9) blood collection points:
   - Administration regimens for 75 mg Q2W and 140 mg Q2W:
      1^{st} administration: before administration (within 1 h); 4 h (± 10 min), 24 h (± 1 h) (day 1), 48 h (± 2 h) (day 2), 72 h (± 3 h) (day 3), 96 h (± 4 h) (day 4), 144 h (± 6 h) (day 6), 168 h (± 6 h) (day 7), 240 h (± 12 h)(day 10) and 288 h (± 12 h) (day 12) after administration;
      2^{nd}/3^{rd}/4^{th}/5^{th} administration: before administration (within 1 h);
      6^{th} administration: before administration (within 1 h); 4 h (± 10 min), 24 h (± 1 h) (day 1), 48 h (± 2 h) (day 2), 72 h (± 3 h) (day 3), 96 h (± 4 h) (day 4), 144 h (± 6 h) (day 6), 168 h (± 6 h) (day 7), 240 h (± 12 h)(day 10), 288 h (± 12 h) (day 12) and 336 h (± 12 h) (day 14) after administration;
      PK/PD (PCSK9) blood samples will be collected in the early termination visit if subjects fail to complete the study and withdraw from the study in advance;
1 PK/PD (PCSK9) blood sample was collected in safety follow-up.
   - Administration regimens for 300 mg Q4W and 420 mg Q4W:
      1^{st} administration: before administration (within 1 h); 4 h (± 10 min), 24 h (± 1 h) (day 1), 48 h (± 2 h) (day 2), 72 h (± 3 h) (day 3), 96 h (± 4 h) (day 4), 144 h (± 6 h) (day 6), 168 h (± 6 h) (day 7), 240 h (± 12 h)(day 10), 336 h (± 12 h) (day 14) and 504 h (± 24 h) (day 21) after administration;
      2^{nd} administration: before administration (within 1 h);
      3^{rd} administration: before administration (within 1 h); 4 h (± 10 min), 24 h (± 1 h) (day 1), 48 h (± 2 h) (day 2), 72 h (± 3 h) (day 3), 96 h (± 4 h) (day 4), 144 h (± 6 h) (day 6), 168 h (± 6 h) (day 7), 240 h (± 12 h)(day 10), 336 h (± 12 h) (day 14), 504 h (± 24 h) (day 21) and 672 h (± 24 h) (day 28) after administration;
      PK/PD blood samples will be collected in the early termination visit if subjects fail to complete the study and withdraw from the study in advance;
1 PK/PD blood sample was collected in safety follow-up.
   Administration regimens for 450 mg Q6W and 600 mg Q6W:
   1^{st} administration: before administration (within 1 h); 4 h (± 10 min), 24 h (± 1 h) (day 1), 48 h (± 2 h) (day 2), 72 h (± 3 h) (day 3), 96 h (± 4 h) (day 4), 144 h (± 6 h) (day 6), 168 h (± 6 h) (day 7), 240 h (± 12 h)(day 10), 336 h (± 12 h) (day 14), 504 h (± 24 h) (day 21), 672 h (± 24 h) (day 28) and 840 h (± 24 h) (day 35) after administration;
   2^{nd} administration: before administration (within 1 h); 4 h (± 10 min), 24 h (± 1 h) (day 1), 48 h (± 2 h) (day 2), 72 h (± 3 h) (day 3), 96 h (± 4 h) (day 4), 144 h (± 6 h) (day 6), 168 h (± 6 h) (day 7), 240 h (± 12 h) (day 10), 336 h (± 12 h) (day 14), 504 h (± 24 h) (day 21), 672 h (± 24 h) (day 28), 840 h (± 24 h) (day 35) and 1008 h (± 48 h )(day 42) after administration;
   The 600 mg dose group collected one PK/PD blood sample at 1344 h (+ 48 h) (day 56) after 2nd administration.
   PK/PD blood samples will be collected in the early termination visit if subjects fail to complete the study and withdraw from the study in advance;
1 PK/PD blood sample was collected in safety follow-up.
   PK/PD (PCSK9) blood samples were detected at a central laboratory. Blood lipids were detected in the local laboratory during the screening period, and detected in the central laboratory from a baseline period.

5 mL of whole blood would be collected using a procoagulant vacuum blood collection tube, and serum was isolated, aliquoted and cryopreserved for PK/PD (PCSK9) analysis.

Blood lipid detection was performed as described above.

### 6. Pharmacokinetic results

Pharmacokinetic parameters for ADI-10087 free concentration were calculated at actual blood collection times using non-compartmental analysis (NCA) using Pkanalix 2019R2 (Lixoft, Antony, France) software.

The summary on the average pharmacokinetic parameters for each dose group at the first administration and administration at homeostasis of ADI-10087 after multiple subcutaneous injections of different doses of ADI-10087 in patients with hypercholesterolemia is shown in Tables 5 and 6.

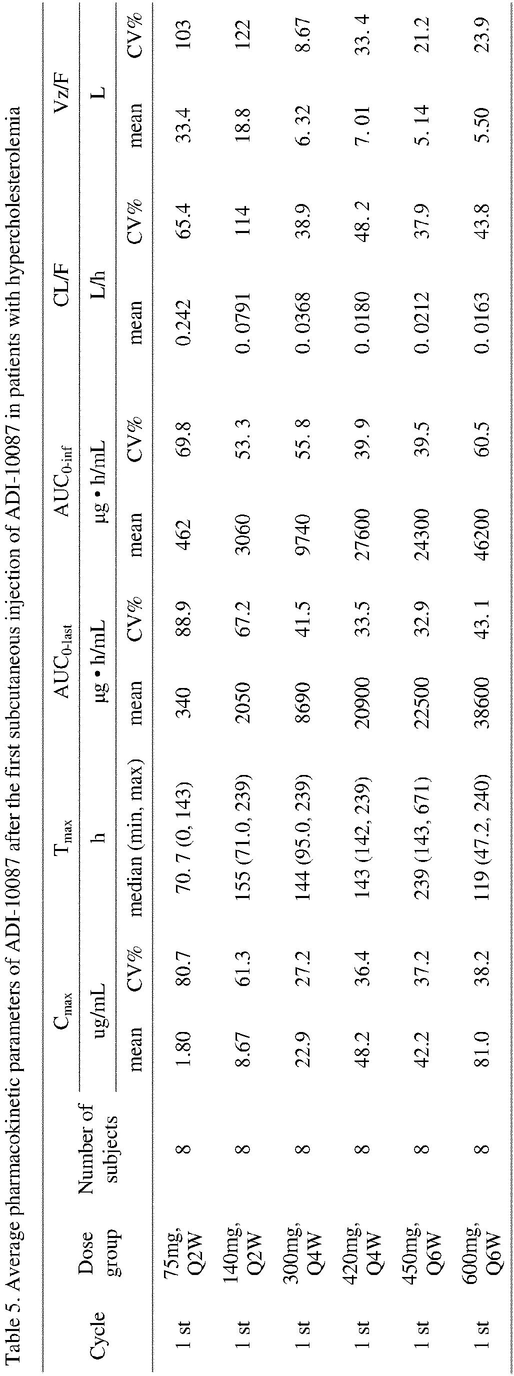

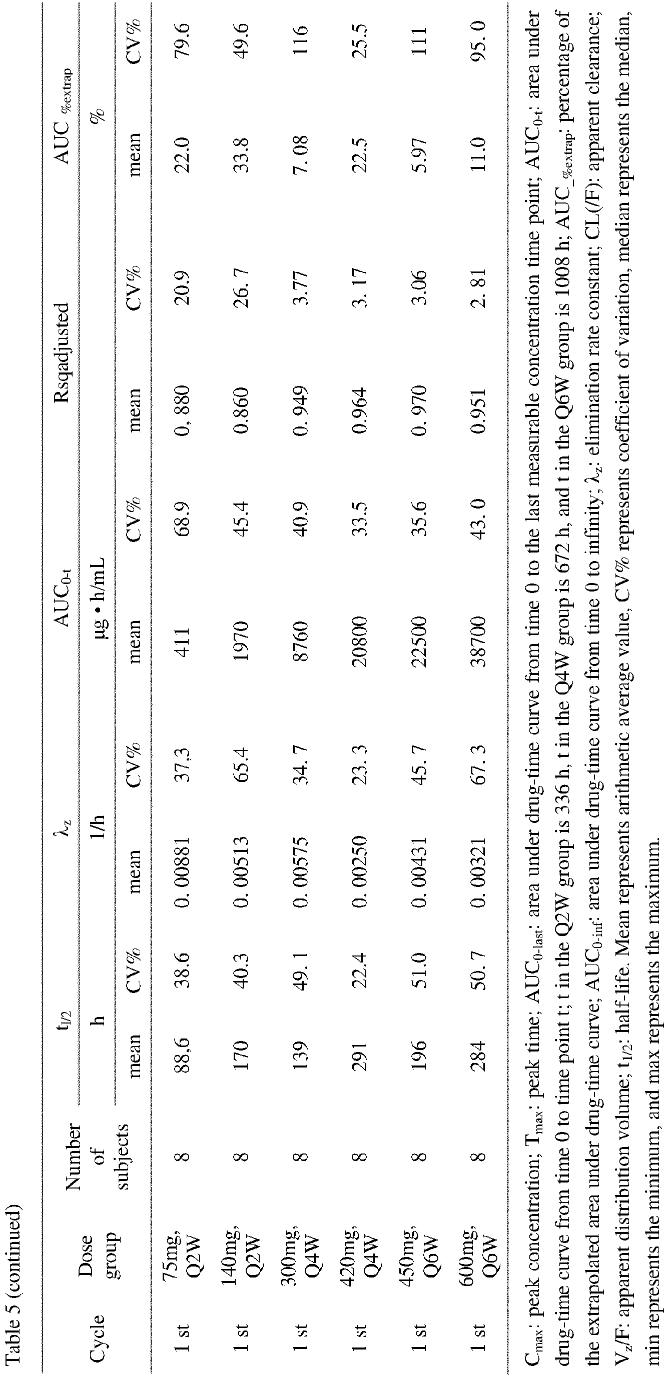

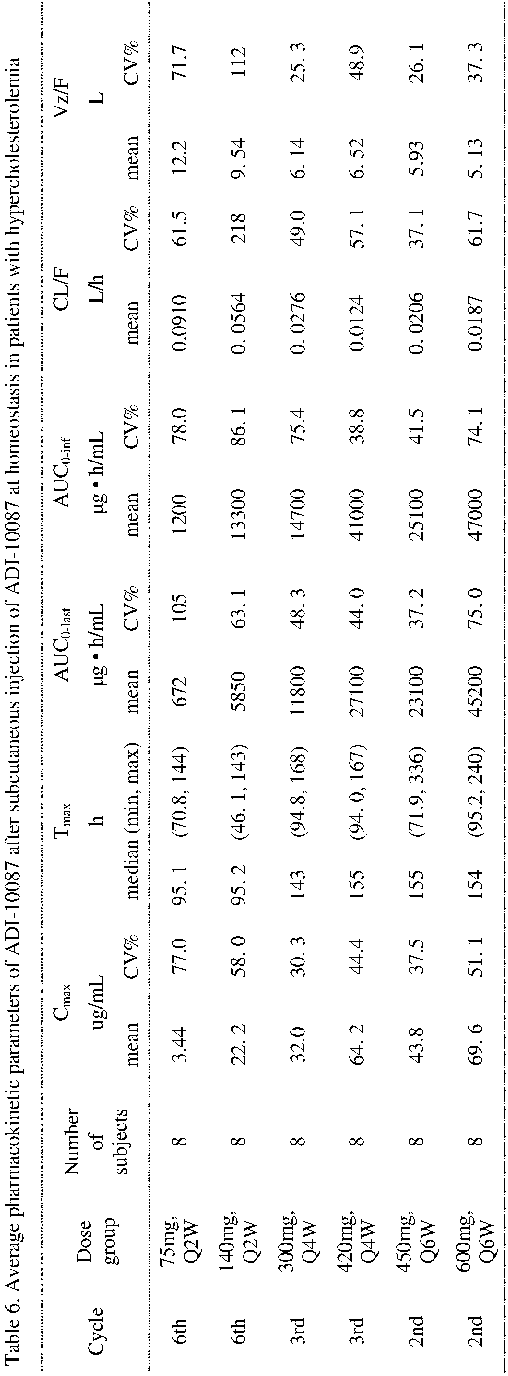

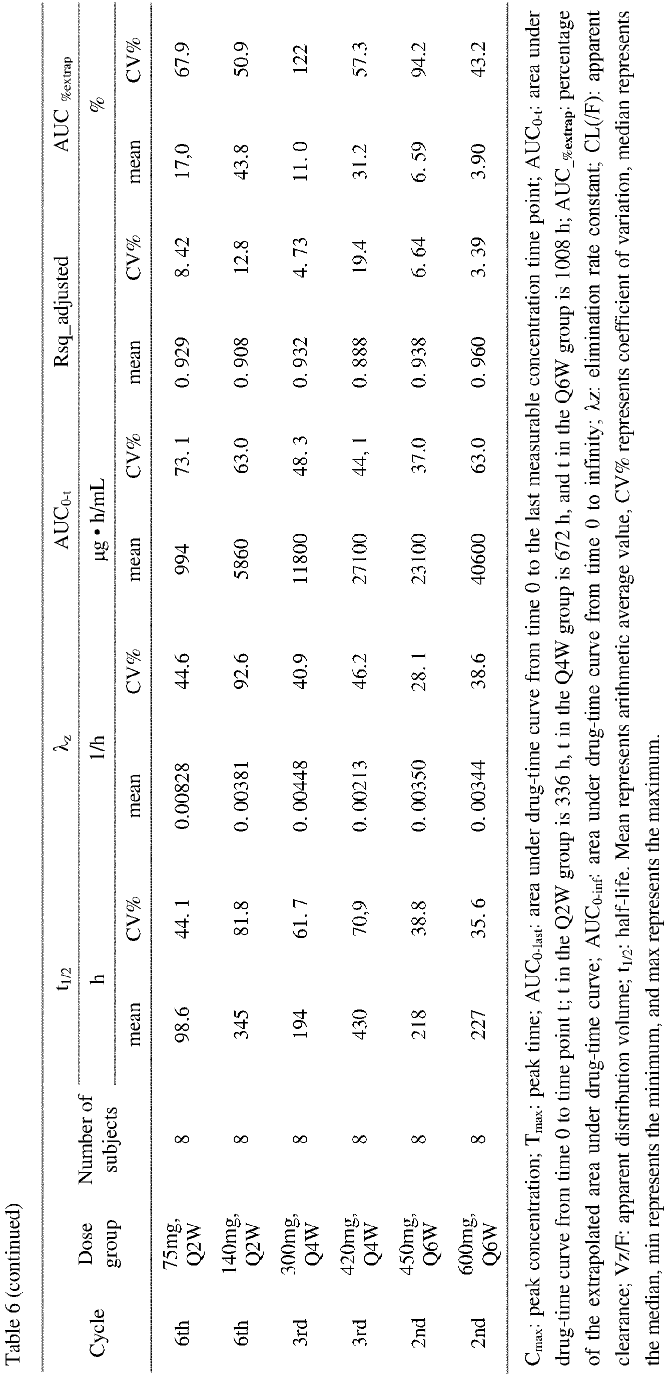

Within a dose range of the first dose of 75-600 mg of ADI-10087, the increase proportion of Cₘₐₓ and AUC₀-_{inf} of ADI-10087 was larger than the increase proportion of the dose, and the non-linear kinetic characteristic was presented.

In cycle 1 and steady-state cycle PK study, after 75-600 mg of ADI-10087 was injected subcutaneously into the abdomen at different administration intervals, the median peak time (Tₘₐₓ) of the ADI-10087 was 70.7-239 h (2.9-10 days); after administration in the homeostasis cycle, after 75-600 mg of ADI-10087 was injected subcutaneously into the abdomen at different administration intervals, the median peak time (Tₘₐₓ) of the ADI-10087 was 95.1-155 h (3.96-6.46 days); the results show that the ADI-10087 needs time for absorption in the abdomen, and when the dose is increased, it needs more time to reach absorption peak.

After 75-600 mg of ADI-10087 was injected subcutaneously into the abdomen at cycle 1, the average Vz/F of the ADI-10087 was about 5.14-33.4 L, the average CL/F was about 0.0163-0.242 L/h, and the average t_{1/2} was about 88.6-291 h (3.69-12.1 days); after 75-600 mg of ADI-10087 was injected subcutaneously into the abdomen in the homeostasis cycle, the average Vz/F of the ADI-10087 was about 5.13-12.2 L, the average CL/F was about 0.0124-0.091 L/h, and the average t_{1/2} was about 98.6-430 h (4.10-17.9 days). As the dose increased, Vz/F and CL/F continuously decreased and t_{1/2} gradually prolonged.

When the drug was administered once every 2 weeks, the mean of accumulation ratios of concentration at 336 h (C₃₃₆ₕ) after ADI-10087 was administered to 75 mg and 140 mg dose groups by multiple subcutaneous injections were 3.44 and 3.83, respectively, and the mean of the accumulation ratios of AUC₀₋₃₃₆ₕ were 3.20 and 3.64, respectively;
when the drug was administered once every 4 weeks, the mean of accumulation ratios of concentration at 672 h (C_{672 h}) after ADI-10087 was administered to 300 mg and 420 mg dose groups by multiple subcutaneous injections were 2.03 and 1.47, respectively, and the mean of the accumulation ratios of AUC₀₋₆₇₂ₕ were 1.34 and 1.28, respectively;
when the drug was administered once every 6 weeks, the mean of accumulation ratios of concentration at 1008 h (C_{1008 h}) after ADI-10087 was administered to 450 mg and 600 mg dose groups by multiple subcutaneous injections were 1.18 and 0.983, respectively, and the mean of the accumulation ratios of AUC₀-_{1008 h} were 1.02 and 0.986, respectively. With dose increased, the administration cycle interval prolonged and the accumulation ratio of ADI-10087 decreased, with results shown in Table 7.

**Table 7. Mean of accumulation ratio (AR) of ADI-10087 after subcutaneous infusion of ADI-10087 at each administration interval**

| Dose group | Administrati on cycle | AR_C₃₃₆ₕ ⁻ | AR_C₆₇₂ₕ ⁻ | AR_C_{1 008h} | AR_AU C₀-₃₃₆ₕ | AR_AU C₀-₆₇₂ₕ | AR_AU C₀-₁₀₀₈ₕ |
|---|---|---|---|---|---|---|---|
| 75mg | Q2W | 3.44 | - | - | 3.20 | - | - |
| 140mg | Q2W | 3.83 | - | - | 3.64 | - | - |
| 300mg | Q4W | - | 2.03 | - | - | 1.34 | - |
| 420mg | Q4W | - | 1.47 | - | - | 1.28 | - |
| 450mg | Q6W | - | - | 1.18 | - | - | 1.02 |
| 600mg | Q6W | - | - | 0.983 | - | - | 0.986 |

### Example 15. Evaluation of Pharmacodynamics of Multiple Dose Repeated Administrations of ADI-10087 in Chinese Population with Hypercholesterolemia

Selection of investigators , design of test, drugs used in the study, administration regimens, and sample collection and process were the same as in Example 14.

### Pharmacodynamic results

Pharmacodynamic evaluation was based on pharmacodynamic analysis set (PDS), changes in serum free PCSK9 concentration relative to baseline before each administration were observed, and the observed values at each time point and the change rate (%) of serum free PCSK9 concentration relative to baseline were summarized. 60 subjects with hypercholesterolemia (48 in the ADI-10087 group and 12 in the placebo group) were included in PDS, PCSK9 data in serum after repeated multiple administrations of ADI-10087 at different administration intervals were analyzed, and PSD included all patient populations who had received at least one study drug and had at least one valid test result after administration.

### 1. Change rate of serum free PCSK9 concentration relative to baseline after administration

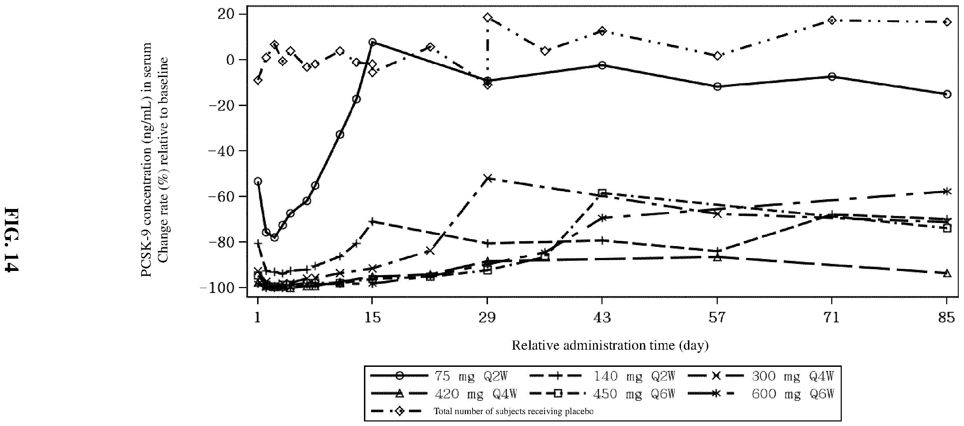

The change rate of serum free PCSK9 concentration relative to baseline after administration is shown in FIG. 14. The results show that: the level of PCSK9 in the placebo group were not significantly changed. After the first administration of ADI-10087 in the ADI-10087 group: PCSK9 in the 75-600 mg dose group basically reached the maximum decrease in about 24 h, and the time to the maximum decrease was consistent in each dose group; after the first administration of ADI-10087, and PCSK9 in the 75 mg dose group reached the maximum decrease of about 78%, and PCSK9 in the 140-600 mg dose group reached the maximum decrease of > 90%; the greater the dose was, the larger the decrease of PCSK9 was, and the PCSK9 was completely suppressed until the dose reached 300 mg or more; after the maximum decrease time, PCSK9 began to rebound, the greater the dose was, the smaller the rebound was. After multiple administrations of ADI-10087, the level of PCSK9 was maintained at a certain decrease level; for the 2-week administration interval cohort, PCSK9 in the 75 mg dose group was maintained at a level comparable to baseline, and PCSK9 in the 140 mg dose group was maintained at a decrease level of about 80%; for the 4-week, 6-week administration interval cohorts, PCSK9 in the 300-600 mg dose group was maintained at a decrease level of 80% or more.

### 2. Comparison of percentage changes of LDL-C relative to baseline at the same administration intervals

2-week administration interval group: the LDL-C level in the 75 mg Q2W group firstly administrated obviously decreased compared with baseline, the maximum decrease before the 2^{nd} administration was about 51.09% (Day 11), there was no obvious rebound between 4 h and 336 h after the last administration, and the LDL-C level could still be greatly decreased (61.323%) compared with baseline after 336 h after the last administration. The LDL-C level in the 140 mg Q2W group also showed a significant decrease after the first administration, with a maximum decrease of about 45.99% before 2^{nd} administration (Day 13). Although rebounded slightly after 4-336 h after the last administration, LDL-C level decreased slightly, and the LDL-C level still decreased by 54.296-60.991% compared with the baseline.

4-week administration interval group: the LDL-C level in the 300 mg Q4W group and the 420 mg Q4W group firstly administrated obviously decreased compared with the baseline, and reached the maximum decrease before the second administration at day 29, which were 59.8% and 71.363% respectively; the decrease of the LDL-C in the 420 mg Q4W group before each administration was obviously larger than that in the 300 mg Q4W group, no obvious rebound was seen in the two groups after the last administration, and the LDL-C in the 450 mg Q4W group could still maintain a decrease of 72.256% (60.136% in the 300 mg Q4W group) after 672 h after the last administration.

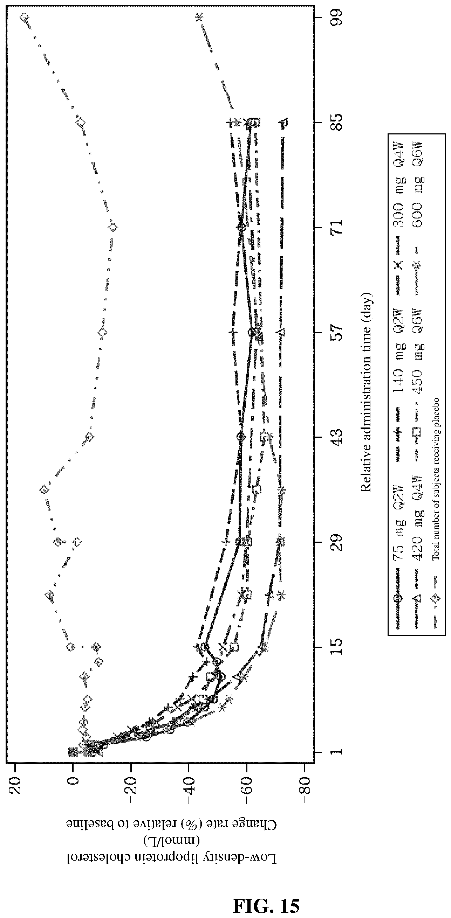

6-week administration interval group: the LDL-C level in the 450 mg Q6W group and the 600 mg Q6W group firstly administrated obviously decreased compared with the baseline, and the decrease in the 600 mg Q6W group was obvious (the maximum decrease was about 71.875%); the decrease of the LDL-C level in the 600 mg Q6W group was still larger than that in the 450 mg Q6W group after the second administration (i.e., after the last administration), and slightly rebounded after 672 h after the second administration, and the decrease was smaller (about 2.9%); the LDL-C level in the 450 mg Q6W and 600 mg Q6W groups still had a better decrease compared with the baseline after 1008 h after the second administration, which were 62.883% and 56.516%, respectively. According to the study results in Example 13, the duration of LDL-C decrease and the duration of PCSK9 inhibition were more obvious at higher doses, while it was observed that the LDL-C level in the 600 mg group still decreased by 43.455% compared with the baseline after 8 weeks (1344 h after the second administration) after administration. It was indicated that ADI-10087 was able to maintain a long interval of 4-8 weeks of administration. The change rate of LDL-C concentration relative to baseline before and after each administration is shown in FIG. 15.

### 3. Change rate of LDL-C level at 6 weeks, 12 weeks, and 14 weeks relative to baseline

After 6 weeks of continuous administration, the LDL-C level significantly decreased relative to baseline in each ADI-10087 dose group compared to placebo, which specifically are: -52.385% in the75 mg Q2W group, -52.523% in the 140 mg Q2W group, -60.353% in the 450 mg Q6W group, and -62.175% in the 600 mg Q6W group; the percentage decrease in LDL-C relative to baseline was dose-dependently increased, i.e., the decrease in LDL-C was more obvious at higher doses.

After 12 weeks of continuous administration, the decrease of the LDL-C level in each dose group of ADI-10087 showed no significant rebound relative to baseline compared with that in 6 weeks after administration, and the percentage changes of LDL-C level relative to baseline in each dose group compared to placebo was: -58.913% in the 75 mg Q2W group, -51.886% in the 140 mg Q2W group, -57.726% in the 300 mg Q4W group, -69.846% in the 420 mg Q4W group, -60.473% in the 450 mg Q6W group, and -54.106% in the 600 mg Q6W group, wherein the decrease of LDL-C level in the 420 mg Q4W group was 72.256% (-2.410% in the placebo group).

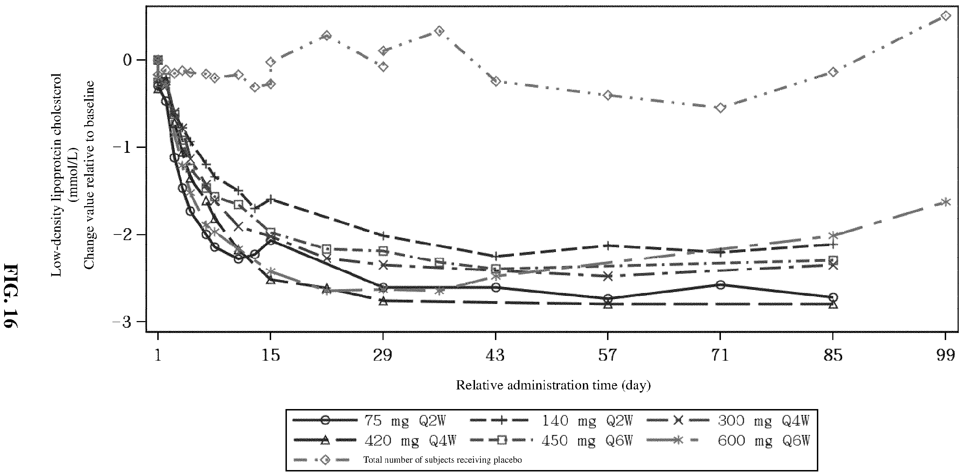

LDL-C level in the 600 mg Q6W group decreased by 43.455% relative to baseline after 14 weeks after administration, i.e., 8 weeks after the second administration (-16.980% in the placebo group). The change rate of LDL-C level relative to baseline before and after each administration is shown in FIG. 16.

### 4. Analysis of change rate (%) of Lp(a) relative to baseline at 6 weeks, 12 weeks and 14 weeks

After 6 weeks of continuous administration, the average decrease of Lp(a) in each ADI-10087 dose group was 8.720% to 22.436% relative to baseline compared with that in the placebo group, with the 75 mg Q2W and 450 mg Q6W groups decreasing most obviously at -22.436% and -21.914%, respectively.

After 12 weeks of continuous administration, the average decrease of Lp(a) in each ADI-10087 dose group was 2.088% to 28.638% relative to baseline compared with that in the placebo group, wherein the decrease was most obvious in the 75 mg Q2W and 450 mg Q6W groups, which were 28.638% and 27.362%, respectively, and the decrease of Lp(a) in the 600 mg Q6W and 420 mg Q4W was smaller, which was 2% to 7%.

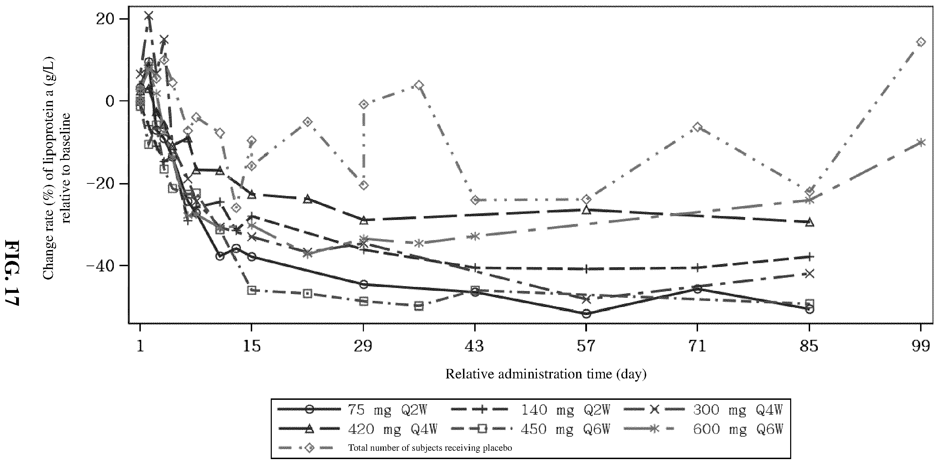

After 14 weeks of continuous administration (8 weeks after the second administration), Lp(a) level in the 600 mg Q6W group decreased by 24.520% relative to baseline was compared with that in the placebo group. The change rate of Lp(a) level relative to baseline before and after each administration is shown in FIG. 17.

### 5. Analysis of change rate (%) of ApoB relative to baseline at 6 weeks, 12 weeks and 14 weeks

After 6 weeks of continuous administration, ApoB showed an obvious decrease relative to baseline compared to placebo. The average decrease of ApoB in each dose group of ADI-100876 was 57.118% to 61.414%; the greater the dose was, the greater the decrease of ApoB was.

After 12 weeks of continuous administration, the average decrease of ApoB in each ADI-10087 dose group was 53.761% to 69.573% relative to baseline compared with that in the placebo group, and ApoB decrease was consistent to LDL-C decrease; wherein the decrease of ApoB in the 420 mg Q4W dose group was close to 70% relative to baseline.

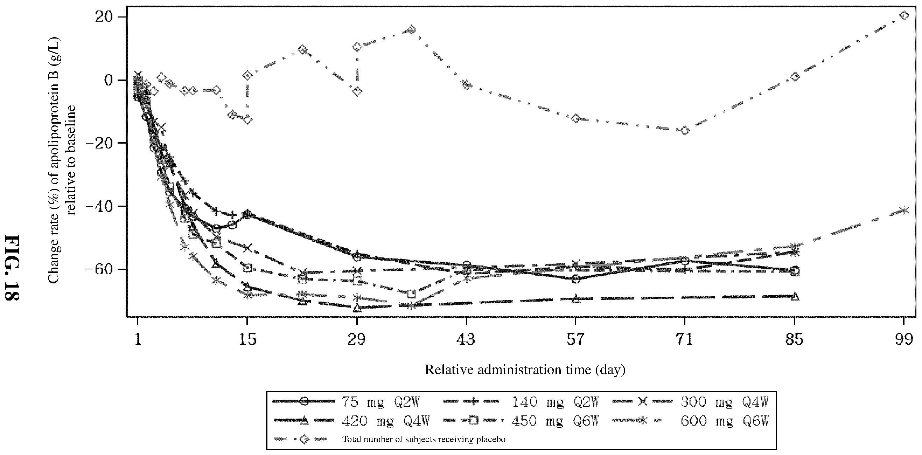

After 14 weeks of continuous administration (8 weeks after the second administration), ApoB level in the 600 mg Q6W group decreased by 61.729% relative to baseline was compared with that in the placebo group. The change rate of ApoB level relative to baseline before and after each administration is shown in FIG. 18.

### 6. Analysis of change rate (%) of non-HDL-C relative to baseline at 12 weeks and 14 weeks

After 12 weeks of continuous administration, the average decrease of non-HDL-C relative to baseline in each ADI-10087 dose group was more than 55% compared with that in the placebo group, and the maximum decrease was 75.394% (in the 420 mg Q4W group);

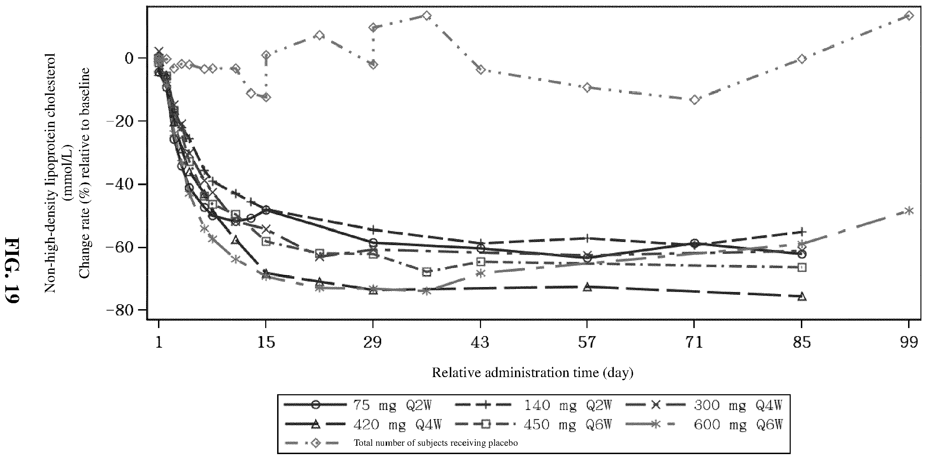

after 14 weeks of continuous administration (8 weeks after the second administration), non-HDL-C level in the 600 mg Q6W group decreased by 61.958% relative to baseline was compared with that in the placebo group. The change rate of non-HDL-C level relative to baseline before and after each administration is shown in FIG. 19.

### 7. Analysis of change rate (%) of TC level relative to baseline at 12 weeks and 14 weeks

After 12 weeks of continuous administration, the average decrease of TC relative to baseline in each ADI-10087 dose groups ranged from 40% to 57%, with the maximum decrease in the 420 mg Q4W group being about 56%. The placebo group had no significant change in TC relative to baseline (increased by about 0.5%).

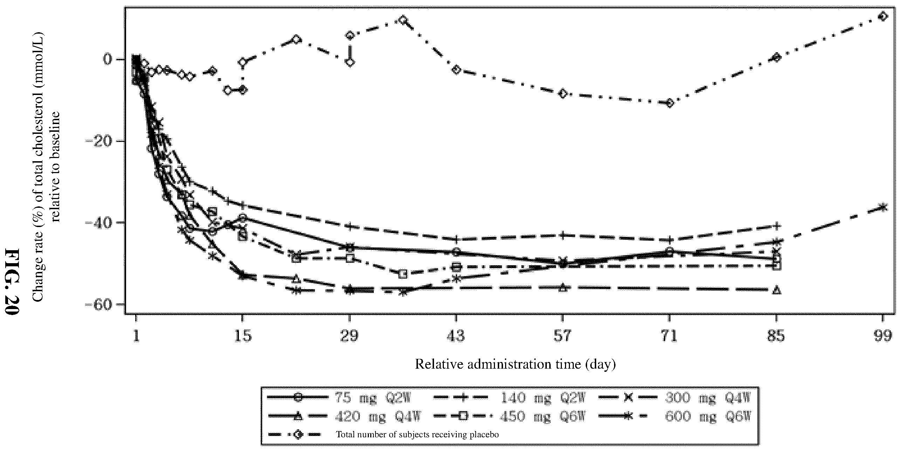

After 14 weeks of continuous administration (8 weeks after the second administration), the TC in the 600 mg Q6W group decreased by 36.184% relative to baseline, while the TC in the placebo group increased by 10.635% relative to baseline. The change rate of TC level relative to baseline before and after each administration is shown in FIG. 20.

### 8. Analysis of change rate (%) of ApoB/ApoAl relative to baseline at 12 weeks and 14 weeks

After 12 weeks of continuous administration, the average decrease of ApoB/ApoAl relative to baseline in each ADI-10087 dose group was more than 50% compared with that in the placebo group, and the maximum decrease was 68.476% (in the 420 mg Q4W group).

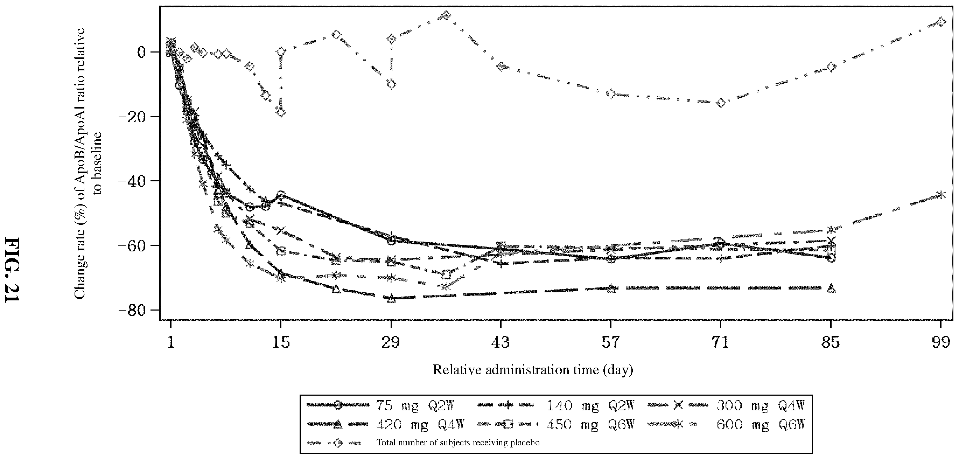

After 14 weeks of continuous administration (8 weeks after the second administration), ApoB/ApoAl level in the 600 mg Q6W group decreased by 53.656% relative to baseline was compared with that in the placebo group. The change rate of ApoB/ApoAl level relative to baseline before and after each administration is shown in FIG. 21.

### 9. Conclusion of efficacy analysis

In the safety analysis set, 48 subjects in the ADI-10087 group and 12 subjects in the placebo group were included in the efficacy analysis. The decrease of average LDL-C concentration in each dose group ranged from 54.3% to 72.26% relative to baseline at week 12. In particular, it was indicated that LDL-C in the 600 mg Q6W cohort decreased by 56.52% (40.54% to 72.50%), which lasted until week 14 (8 weeks after the last administration) and still had a decrease of 43.46% (25.96% to 60.96%) compared with baseline. The average decrease of Lp(a) at week 12 compared with baseline ranged from 24.04% to 50.59%. Other lipids in each dose group also decreased compared with that in the placebo group. Therefore, the PK/PD (LDL-C) of the ADI-10087 is good in characteristic, and can meet the requirement of long-interval administration of 4-8 weeks.

### Example 16. Evaluation of Safety of Multiple Dose Repeated Administrations of ADI-10087 in Chinese Population with Hypercholesterolemia

The results show that: multiple dose repeated subcutaneous injections of ADI-10087 in this study are of good safety in Chinese population with hypercholesterolemia. The incidence rate of treatment emergent adverse events (TEAEs) is 70.8% and the incidence rate of drug-related TEAEs is 31.3% in the ADI-10087 group; the incidence rate of TEAEs is 75.0% and the incidence rate of drug-related TEAEs is 41.7% in the placebo group. The incidence rate of at least one adverse event of specific interest (allergic reaction, injection site reaction, transaminases increased, liver damage and muscle event) in the ADI-10087 and placebo groups were 10.4% and 25.0%, respectively, without severe adverse events, drug-related severe adverse events, adverse events leading to withdrawal from study and adverse events leading to death occurring. The drug-related adverse events in the ADI-10087 group were mild to moderate without special treatment and improved after corrective treatment, which has good safety. Adverse events during the study are summarized in Table 8 below.

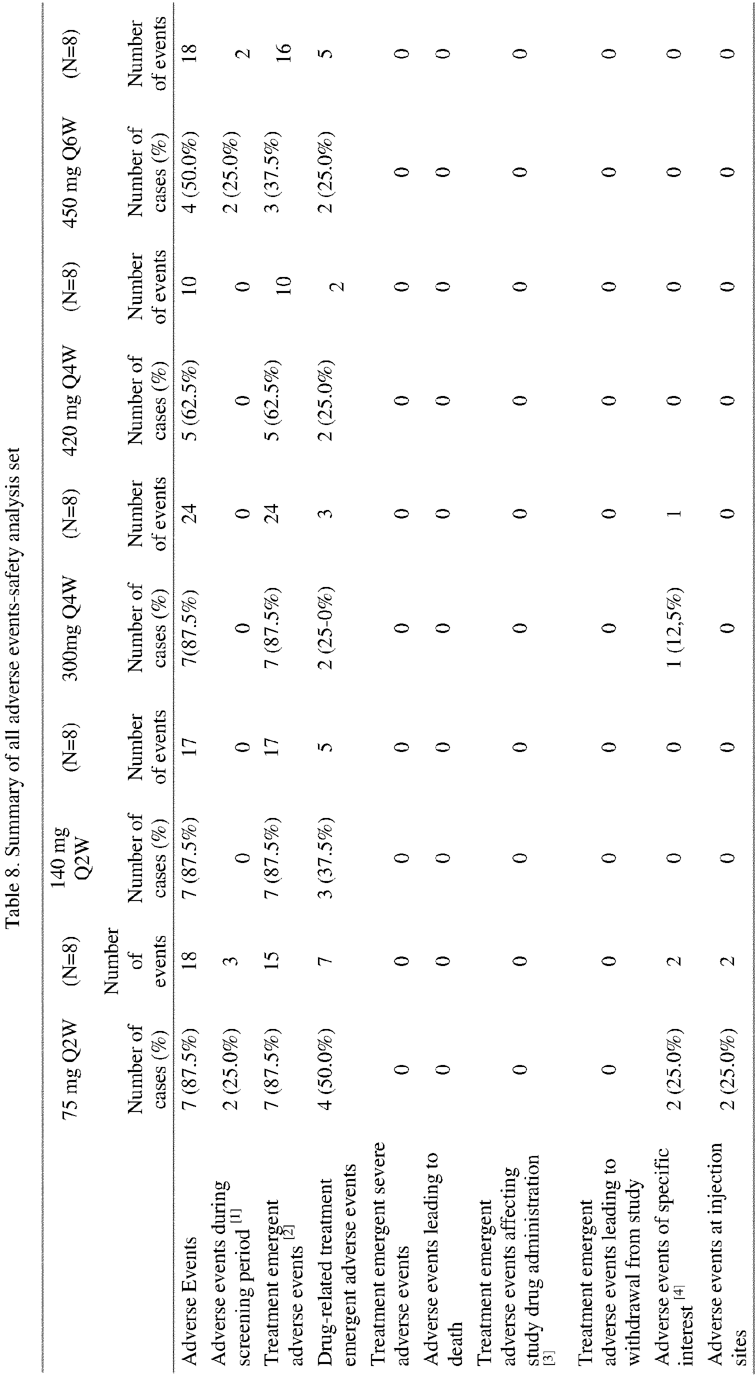

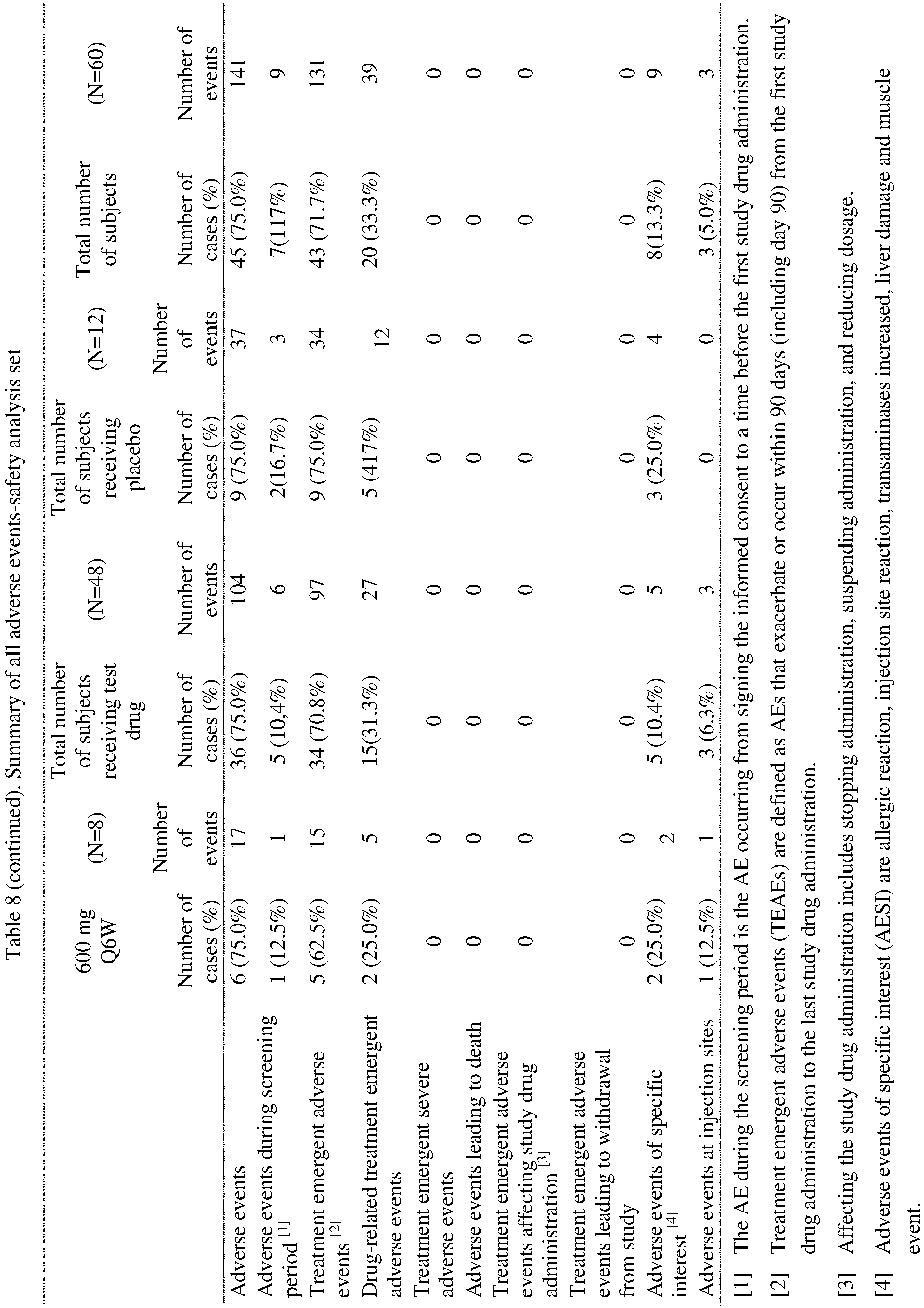

The exemplary embodiments of the present invention have been described above. It should be understood by those skilled in the art that these contents are merely exemplary, and various other replacements, adaptations and modifications can be made within the scope of the present invention. Accordingly, the present invention is not limited to the specific embodiments listed herein.

## Claims

1. A method for lowering cholesterol level in a subject, comprising: administering to the subject an anti-PCSK9 antibody or antigen-binding fragment thereof at a dose of about 15 mg to 3500 mg.

2. A method for preventing or treating a disorder related to increased LDL-cholesterol level in a subject, comprising: administering to the subject an anti-PCSK9 antibody or antigen-binding fragment thereof at a dose of about 15 mg to 3500 mg.

3. A method for preventing or treating a cholesterol-related disease, such as hypercholesterolemia and/or hyperlipidemia, comprising administering to a subject an anti-PCSK9 antibody or antigen-binding fragment thereof at a dose of about 15 mg to 3500 mg.

4. The method according to any one of claims 1-3, wherein the anti-PCSK9 antibody or antigen-binding fragment thereof comprises a heavy chain variable region VH and a light chain variable region VL, wherein
(a) the VH comprises:
(i) a combination of HCDR1, HCDR2 and HCDR3, wherein HCDR1 comprises or consists of an amino acid sequence set forth in SEQ ID NO: 10; HCDR2 comprises or consists of an amino acid sequence set forth in SEQ ID NO: 17; HCDR3 comprises or consists of an amino acid sequence set forth in SEQ ID NO: 19; or
(ii) three complementarity determining regions (CDRs) contained in a VH set forth in SEQ ID NO: 30; and
(b) the VL comprises:
(i) a combination of LCDR1, LCDR2 and LCDR3, wherein LCDR1 comprises or consists of an amino acid sequence set forth in SEQ ID NO: 4; LCDR2 comprises or consists of an amino acid sequence set forth in SEQ ID NO: 5; LCDR3 comprises or consists of an amino acid sequence set forth in SEQ ID NO: 6; or
(ii) three complementarity determining regions (CDRs) contained in a VL set forth in SEQ ID NO: 24.

5. The method according to any one of claims 1-4, wherein the anti-PCSK9 antibody or antigen-binding fragment thereof comprises a heavy chain variable region VH and a light chain variable region VL, wherein
(a) the heavy chain variable region VH comprises or consists of an amino acid sequence set forth in SEQ ID NO: 30; and
(b) the light chain variable region VL comprises or consists of an amino acid sequence set forth in SEQ ID NO: 24.

6. The method according to any one of claims 1-5, wherein the anti-PCSK9 antibody or antigen-binding fragment thereof comprises a heavy chain and a light chain, wherein
(a) the heavy chain comprises or consists of an amino acid sequence set forth in SEQ ID NO: 41; and
(b) the light chain comprises or consists of an amino acid sequence set forth in SEQ ID NO: 35; preferably, the anti-PCSK9 antibody is ADI-10087.

7. The method according to any one of claims 1-6, wherein the cholesterol is LDL-cholesterol.

8. The method according to any one of claims 1-7, wherein the anti-PCSK9 antibody or antigen-binding fragment thereof is administered to a subject at a dose of about 25 mg to about 3000 mg, about 50 mg to about 2500 mg, about 75 mg to about 2000 mg, about 100 mg to about 2000 mg, about 200 mg to about 2000 mg, about 250 mg to about 1500 mg, about 300 mg to about 1000 mg, about 450 mg to about 1000 mg, about 600 mg to about 1000 mg, about 350 mg to about 900 mg, about 400 mg to about 800 mg, about 450 mg to about 700 mg, about 300 mg to about 600 mg, or about 450 mg to about 600 mg, preferably 450 mg or 600 mg.

9. The method according to any one of claims 1-8, wherein the anti-PCSK9 antibody or antigen-binding fragment thereof is administered parenterally, e.g., subcutaneously or intravenously.

10. The method according to any one of claims 1-9, wherein an LDL-cholesterol level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the LDL-cholesterol level in the subject before administration.

11. The method according to any one of claims 1-10, wherein a serum free PCSK-9 level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the serum free PCSK-9 level in the subject before administration.

12. The method according to any one of claims 1-11, wherein a total cholesterol level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the total cholesterol level in the subject before administration.

13. The method according to any one of claims 1-12, wherein an apolipoprotein B level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the apolipoprotein B level in the subject before administration.

14. The method according to any one of claims 1-13, wherein a non-high-density lipoprotein cholesterol level in a subject is still decreased by > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the non-high-density lipoprotein cholesterol level in the subject before administration.

15. The method according to any one of claims 1-14, wherein a lipoprotein a level in a subject is still decreased by > 20%, > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the lipoprotein a level in the subject before administration.

16. The method according to any one of claims 1-15, wherein an ApoB/ApoAl level in a subject is still decreased by > 20%, > 30%, > 35%, > 40%, > 45%, > 50%, > 55%, > 60%, > 65%, > 70%, > 75%, > 80%, > 85%, or > 90% at 4 weeks after administration, 5 weeks after administration, 6 weeks after administration, 7 weeks after administration, 8 weeks after administration, 9 weeks after administration, 10 weeks after administration, 3 months after administration, 4 months after administration or 5 months after administration compared to the ApoB/ApoAl level in the subject before administration.

17. The method according to any one of claims 1-16, wherein the cholesterol or the LDL-cholesterol is serum LDL-cholesterol.

18. The method according to any one of claims 1-17, wherein the anti-PCSK9 antibody or antigen-binding fragment thereof is administered once at an interval equal to or greater than every four weeks (Q4W), e.g., once every four weeks (Q4W), once every five weeks (Q5W), once every six weeks (Q6W), once every seven weeks (Q7W), once every eight weeks (Q8W), once every nine weeks (Q9W), once every ten weeks (Q10W), once every eleven weeks (Q11W), once every twelve weeks (Q12W), once every 4 months, once every 5 months, once every 6 months, once every 7 months, or once a year, preferably once every four weeks (Q4W), once every six weeks (Q6W), once every eight weeks (Q8W).

19. The method according to any one of claims 1-18, wherein the subject, after administration, does not develop severe adverse events, particularly severe adverse events associated with the anti-PCSK9 antibody or antigen-binding fragment thereof.

20. The method according to any one of claims 1-19, wherein the subject has an incidence of adverse events comparable to a subject receiving placebo after administration.

21. The method according to any one of claims 1-20, wherein the cholesterol-related disease is selected from homozygous familial hypercholesterolemia, heterozygous familial hypercholesterolemia and non-familial hypercholesterolemia.

22. The method according to any one of claims 1-21, wherein the anti-PCSK9 antibody or antigen-binding fragment thereof is administered from a liquid antibody preparation comprising:
(i) about 100 mg/mL to about 200 mg/mL of the anti-PCSK-9 antibody or antigen-binding fragment thereof;
(ii) about 0.2 mg/mL to 10 mg/mL of histidine;
(iii) about 1% to 6% of sorbitol and/or about 50 mmol/L to 180 mmol/L of arginine or arginine salt; and
(iv) about 0.05 mg/mL to 1 mg/mL of polysorbate-80 or polysorbate-20;
wherein the pH of the liquid preparation is about 5.0 to 6.0.

23. The method according to any one of claims 1-22, wherein the anti-PCSK9 antibody or antigen-binding fragment thereof is administered from a liquid antibody preparation, and the liquid antibody preparation uses water as a solvent and consists of the following compositions:
| | |
|---|---|
| Anti-PCSK9 antibody or antigen-binding fragment thereof | 150 mg/mL |
| Histidine | 1.5 g/L |
| Arginine | 90 mmol/L |
| Sorbitol | 3% (w/v) |
| Polysorbate 80 | 0.3 g/L; |
wherein the pH of the liquid preparation is about 5.5.

24. A single pharmaceutical dosage unit, comprising an anti-PCSK9 antibody or antigen-binding fragment thereof at a dose of about 15 mg to 3500 mg; preferably, an anti-PCSK9 antibody or antigen-binding fragment thereof at a dose of about 25 mg to about 3000 mg, about 50 mg to about 2500 mg, about 75 mg to about 2000 mg, about 100 mg to about 2000 mg, about 200 mg to about 2000 mg, about 250 mg to about 1500 mg, about 300 mg to about 1000 mg, about 450 mg to about 1000 mg, about 600 mg to about 1000 mg, about 350 mg to about 900 mg, about 400 mg to about 800 mg, about 450 mg to about 700 mg, about 300 mg to about 600 mg, or about 450 mg to about 600 mg; more preferably, an anti-PCSK9 antibody or antigen-binding fragment thereof at a dose of 450 mg or 600 mg.

25. The single pharmaceutical dosage unit according to claim 24, wherein the anti-PCSK9 antibody or antigen-binding fragment thereof comprises a heavy chain variable region VH and a light chain variable region VL, wherein
(a) the VH comprises:
(i) a combination of HCDR1, HCDR2 and HCDR3, wherein HCDR1 comprises or consists of an amino acid sequence set forth in SEQ ID NO: 10; HCDR2 comprises or consists of an amino acid sequence set forth in SEQ ID NO: 17; HCDR3 comprises or consists of an amino acid sequence set forth in SEQ ID NO: 19; or
(ii) three complementarity determining regions (CDRs) contained in a VH set forth in SEQ ID NO: 30; and
(b) the VL comprises:
(i) a combination of LCDR1, LCDR2 and LCDR3, wherein LCDR1 comprises or consists of an amino acid sequence set forth in SEQ ID NO: 4; LCDR2 comprises or consists of an amino acid sequence set forth in SEQ ID NO: 5; LCDR3 comprises or consists of an amino acid sequence set forth in SEQ ID NO: 6; or
(ii) three complementarity determining regions (CDRs) contained in a VL set forth in SEQ ID NO: 24.

26. The single pharmaceutical dosage unit according to claim 25, wherein the anti-PCSK9 antibody or antigen-binding fragment thereof comprises a heavy chain variable region VH and a light chain variable region VL, wherein,
(a) the heavy chain variable region VH comprises or consists of an amino acid sequence set forth in SEQ ID NO: 30; and
(b) the light chain variable region VL comprises or consists of an amino acid sequence set forth in SEQ ID NO: 24.

27. The single pharmaceutical dosage unit according to claim 26, wherein the anti-PCSK9 antibody or antigen-binding fragment thereof comprises a heavy chain and a light chain, wherein
(a) the heavy chain comprises or consists of an amino acid sequence set forth in SEQ ID NO: 41; and
(b) the light chain comprises or consists of an amino acid sequence set forth in SEQ ID NO: 35;
preferably, the anti-PCSK9 antibody is ADI-10087.

28. A pharmaceutical kit, comprising the anti-PCSK9 antibody or antigen-binding fragment thereof according to any one of claims 24-27.

29. Use of the single pharmaceutical dosage unit according to any one of claims 24-27 or the kit of parts according to claim 28 in preparing a medicament for lowering cholesterol level in a subject.

30. Use of the single pharmaceutical dosage unit according to any one of claims 24-27 or the kit of parts according to claim 28 in preparing a medicament for the prevention or treatment of a disorder associated with increased LDL-cholesterol level in a subject.

31. Use of the single pharmaceutical dosage unit according to any one of claims 24-27 or the kit of parts according to claim 28 in preparing a medicament for the prevention or treatment of a cholesterol-related disease such as hypercholesterolemia and/or hyperlipidemia.
